(19) Europäisches Patentamt
European Patent Office
Office européen des brevets

(11) **EP 2 305 309 A2**

(12) **EUROPEAN PATENT APPLICATION**
published in accordance with Art. 153(4) EPC

(43) Date of publication:
**06.04.2011 Bulletin 2011/14**

(51) Int Cl.:
***A61K 47/48*** *(2006.01)*

(21) Application number: **09761841.7**

(22) Date of filing: **12.06.2009**

(86) International application number:
**PCT/ES2009/070224**

(87) International publication number:
**WO 2009/150284 (17.12.2009 Gazette 2009/51)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **13.06.2008 ES 200801796**

(71) Applicant: **Proyecto de Biomedicina Cima, S.L.**
**31008 Pamplona - Navarra (ES)**

(72) Inventors:
• **PRIETO VALTUEÑA, Jesús María**
  **E-31008 Pamplona - Navarra (ES)**
• **BERRAONDO LÓPEZ, Pedro**
  **E-31008 Pamplona - Navarra (ES)**
• **FIORAVANTI, Jessica**
  **E-31008 Pamplona - Navarra (ES)**

(74) Representative: **ABG Patentes, S.L.**
**Avenida de Burgos 16D**
**Edificio Euromor**
**28036 Madrid (ES)**

(54) **CONJUGATES FOR THE ADMINISTRATION OF BIOLOGICALLY ACTIVE COMPOUNDS**

(57)     The invention relates to a conjugate that comprises an Apo A molecule or a functionally equivalent variant thereof and a compound of therapeutic interest wherein both components are covalently coupled as well as to the use of said conjugates in therapy for the specific targeting of said compounds to those tissues showing specific binding sites for the ApoA molecule.

EP 2 305 309 A2

SEQUENCE LISTING

<110> PROYECTO DE BIOMEDICINA CIMA S.L.

<120> CONJUGATES FOR THE ADMINISTRATION OF BIOLOGICALLY ACTIVE COMPOUNDS

<130> P3764PC00

<150> ES P200801796
<151> 2008-06-13

<160> 54

<170> PatentIn version 3.4

<210> 1
<211> 267
<212> PRT
<213> Homo sapiens

<400> 1

Met Lys Ala Ala Val Leu Thr Leu Ala Val Leu Phe Leu Thr Gly Ser
1               5                   10                  15

Gln Ala Arg His Phe Trp Gln Gln Asp Glu Pro Pro Gln Ser Pro Trp
            20                  25                  30

Asp Arg Val Lys Asp Leu Ala Thr Val Tyr Val Asp Val Leu Lys Asp
            35                  40                  45

Ser Gly Arg Asp Tyr Val Ser Gln Phe Glu Gly Ser Ala Leu Gly Lys
        50                  55                  60

Gln Leu Asn Leu Lys Leu Leu Asp Asn Trp Asp Ser Val Thr Ser Thr
65                  70                  75                  80

Phe Ser Lys Leu Arg Glu Gln Leu Gly Pro Val Thr Gln Glu Phe Trp
                85                  90                  95

Asp Asn Leu Glu Lys Glu Thr Glu Gly Leu Arg Gln Glu Met Ser Lys
            100                 105                 110

Asp Leu Glu Glu Val Lys Ala Lys Val Gln Pro Tyr Leu Asp Asp Phe
            115                 120                 125

Gln Lys Lys Trp Gln Glu Glu Met Glu Leu Tyr Arg Gln Lys Val Glu
        130                 135                 140

Pro Leu Arg Ala Glu Leu Gln Glu Gly Ala Arg Gln Lys Leu His Glu
145                 150                 155                 160

```
Leu Gln Glu Lys Leu Ser Pro Leu Gly Glu Glu Met Arg Asp Arg Ala
                165             170             175

Arg Ala His Val Asp Ala Leu Arg Thr His Leu Ala Pro Tyr Ser Asp
            180             185             190

Glu Leu Arg Gln Arg Leu Ala Ala Arg Leu Glu Ala Leu Lys Glu Asn
            195             200             205

Gly Gly Ala Arg Leu Ala Glu Tyr His Ala Lys Ala Thr Glu His Leu
        210             215             220

Ser Thr Leu Ser Glu Lys Ala Lys Pro Ala Leu Glu Asp Leu Arg Gln
225             230             235             240

Gly Leu Leu Pro Val Leu Glu Ser Phe Lys Val Ser Phe Leu Ser Ala
                245             250             255

Leu Glu Glu Tyr Thr Lys Lys Leu Asn Thr Gln
                260             265
```

```
<210>  2
<211>  264
<212>  PRT
<213>  Mus musculus

<400>  2
```

```
Met Lys Ala Val Val Leu Ala Val Ala Leu Val Phe Leu Thr Gly Ser
1               5               10              15

Gln Ala Trp His Val Trp Gln Gln Asp Glu Pro Gln Ser Gln Trp Asp
            20              25              30

Lys Val Lys Asp Phe Ala Asn Val Tyr Val Asp Ala Val Lys Asp Ser
        35              40              45

Gly Arg Asp Tyr Val Ser Gln Phe Glu Ser Ser Ser Leu Gly Gln Gln
        50              55              60

Leu Asn Leu Asn Leu Leu Glu Asn Trp Asp Thr Leu Gly Ser Thr Val
65              70              75              80

Ser Gln Leu Gln Glu Arg Leu Gly Pro Leu Thr Arg Asp Phe Trp Asp
                85              90              95
```

**33**

```
Asn Leu Glu Lys Glu Thr Asp Trp Val Arg Gln Glu Met Asn Lys Asp
            100                 105                 110

Leu Glu Glu Val Lys Gln Lys Val Gln Pro Tyr Leu Asp Glu Phe Gln
            115                 120                 125

Lys Lys Trp Lys Glu Asp Val Glu Leu Tyr Arg Gln Lys Val Ala Pro
            130                 135                 140

Leu Gly Ala Glu Leu Gln Glu Ser Ala Arg Gln Lys Leu Gln Glu Leu
145                 150                 155                 160

Gln Gly Arg Leu Ser Pro Val Ala Glu Glu Phe Arg Asp Arg Met Arg
                165                 170                 175

Thr His Val Asp Ser Leu Arg Thr Gln Leu Ala Pro His Ser Glu Gln
            180                 185                 190

Met Arg Glu Ser Leu Ala Gln Arg Leu Ala Glu Leu Lys Ser Asn Pro
            195                 200                 205

Thr Leu Asn Glu Tyr His Thr Arg Ala Lys Thr His Leu Lys Thr Leu
    210                 215                 220

Gly Glu Lys Ala Arg Pro Ala Leu Glu Asp Leu Arg His Ser Leu Met
225                 230                 235                 240

Pro Met Leu Glu Thr Leu Lys Thr Lys Ala Gln Ser Val Ile Asp Lys
                245                 250                 255

Ala Ser Glu Thr Leu Thr Ala Gln
                260


<210>  3
<211>  259
<212>  PRT
<213>  Rattus norvegicus

<400>  3

Met Lys Ala Ala Val Leu Ala Val Ala Leu Val Phe Leu Thr Gly Cys
1               5                   10                  15

Gln Ala Trp Glu Phe Trp Gln Gln Asp Glu Pro Gln Ser Gln Trp Asp
                20                  25                  30
```

34

Arg Val Lys Asp Phe Ala Thr Val Tyr Val Asp Ala Val Lys Asp Ser
        35                  40                  45

Gly Arg Asp Tyr Val Ser Gln Phe Glu Ser Ser Thr Leu Gly Lys Gln
        50                  55                  60

Leu Asn Leu Asn Leu Leu Asp Asn Trp Asp Thr Leu Gly Ser Thr Val
65                  70                  75                  80

Gly Arg Leu Gln Glu Gln Leu Gly Pro Val Thr Gln Glu Phe Trp Ala
                85                  90                  95

Asn Leu Glu Lys Glu Thr Asp Trp Leu Arg Asn Glu Met Asn Lys Asp
                100                 105                 110

Leu Glu Asn Val Lys Gln Lys Met Gln Pro His Leu Asp Glu Phe Gln
                115                 120                 125

Glu Lys Trp Asn Glu Glu Val Glu Ala Tyr Arg Gln Lys Leu Glu Pro
        130                 135                 140

Leu Gly Thr Glu Leu His Lys Asn Ala Lys Glu Met Gln Arg His Leu
145                 150                 155                 160

Lys Val Val Ala Glu Glu Phe Arg Asp Arg Met Arg Val Asn Ala Asp
                165                 170                 175

Ala Leu Arg Ala Lys Phe Gly Leu Tyr Ser Asp Gln Met Arg Glu Asn
                180                 185                 190

Leu Ala Gln Arg Leu Thr Glu Ile Lys Asn His Pro Thr Leu Ile Glu
        195                 200                 205

Tyr His Thr Lys Ala Ser Asp His Leu Lys Thr Leu Gly Glu Lys Ala
        210                 215                 220

Lys Pro Ala Leu Asp Asp Leu Gly Gln Gly Leu Met Pro Val Leu Glu
225                 230                 235                 240

Ala Trp Lys Ala Lys Ile Met Ser Met Ile Asp Glu Ala Lys Lys Lys
                245                 250                 255

Leu Asn Ala

```
<210>   4
<211>   14
<212>   PRT
<213>   Artificial sequence

<220>
<223>   TGF-beta1 inhibitor peptide p144

<400>   4

Thr Ser Leu Asp Ala Ser Ile Ile Trp Ala Met Met Gln Asn
1               5                   10


<210>   5
<211>   14
<212>   PRT
<213>   Artificial sequence

<220>
<223>   TGF-beta1 inhibitor peptide p17

<400>   5

Thr Ser Leu Asp Ala Ser Ile Ile Trp Ala Met Met Gln Asn
1               5                   10


<210>   6
<211>   14
<212>   PRT
<213>   Artificial sequence

<220>
<223>   Linker rich in Gly 1

<400>   6

Ser Gly Gly Thr Ser Gly Ser Thr Ser Gly Thr Gly Ser Thr
1               5                   10


<210>   7
<211>   15
<212>   PRT
<213>   Artificial sequence

<220>
<223>   Linker rich in Gly 2

<400>   7

Ala Gly Ser Ser Thr Gly Ser Ser Thr Gly Pro Gly Ser Thr Thr
1               5                   10                  15


<210>   8
<211>   7
<212>   PRT
<213>   Artificial sequence
```

```
<220>
<223>  Linker rich in Gly 3

<400>  8

Gly Gly Ser Gly Gly Ala Pro
1               5


<210>  9
<211>  8
<212>  PRT
<213>  Artificial sequence

<220>
<223>  Linker rich in Gly 4

<400>  9

Gly Gly Gly Val Glu Gly Gly Gly
1               5


<210>  10
<211>  7
<212>  PRT
<213>  Artificial sequence

<220>
<223>  Tetranectin trimerization sequence

<400>  10

Gly Thr Lys Val His Met Lys
1               5


<210>  11
<211>  13
<212>  PRT
<213>  Artificial sequence

<220>
<223>  Fibronectin linker

<400>  11

Pro Gly Thr Ser Gly Gln Gln Pro Ser Val Gly Gln Gln
1               5                   10


<210>  12
<211>  10
<212>  PRT
<213>  Artificial sequence

<220>
<223>  Linker derived from IgG3
```

```
<400>  12

Pro Lys Pro Ser Thr Pro Pro Gly Ser Ser
1               5                   10


<210>  13
<211>  11
<212>  PRT
<213>  Artificial sequence

<220>
<223>  Linker

<400>  13

Ala Pro Ala Glu Thr Lys Ala Glu Pro Met Thr
1               5                   10


<210>  14
<211>  5
<212>  PRT
<213>  Artificial sequence

<220>
<223>  Enterokinase cleavage site

<400>  14

Asp Asp Asp Asp Lys
1               5


<210>  15
<211>  5
<212>  PRT
<213>  Artificial sequence

<220>
<223>  Factor Xa cleavage site

<400>  15

Ile Glu Asp Gly Arg
1               5


<210>  16
<211>  6
<212>  PRT
<213>  Artificial sequence

<220>
<223>  Thrombin cleavage site

<400>  16

Leu Val Pro Arg Gly Ser
1               5
```

```
<210>  17
<211>  7
<212>  PRT
<213>  Artificial sequence

<220>
<223>  TEV protease cleavage site

<400>  17

Glu Asn Leu Tyr Phe Gln Gly
1               5


<210>  18
<211>  8
<212>  PRT
<213>  Artificial sequence

<220>                      .
<223>  PreScission protease cleavage site

<400>  18

Leu Glu Val Leu Phe Gln Gly Pro
1               5


<210>  19
<211>  5
<212>  PRT
<213>  Artificial sequence

<220>
<223>  MMP9 protease cleavage site

<400>  19

Leu Phe Pro Thr Ser
1               5


<210>  20
<211>  20
<212>  DNA
<213>  Artificial sequence

<220>
<223>  Primer FwATGmApoA1

<400>  20
atgaaagctg tggtgctggc                                       20


<210>  21
<211>  20
<212>  DNA
<213>  Artificial sequence
```

```
<220>
<223>   Primer RvTGAmApoA1

<400>   21
tcactgggca gtcagagtct                                          20


<210>   22
<211>   20
<212>   DNA
<213>   Artificial sequence

<220>
<223>   Primer FwATGmIFNalpha1

<400>   22
atggctaggc tctgtgcttt                                          20


<210>   23
<211>   20
<212>   DNA
<213>   Artificial sequence

<220>
<223>   Primer RvTGAmIFNalpha1

<400>   23
tcatttctct tctctcagtc                                          20


<210>   24
<211>   28
<212>   DNA
<213>   Artificial sequence

<220>
<223>   Primer RvAscImApoA1                         .

<400>   24
ggcgcgccct gggcagtcag agtctcgc                                 28


<210>   25
<211>   29
<212>   DNA
<213>   Artificial sequence

<220>
<223>   Primer FwAscImIFNalpha1

<400>   25
ggcgcgccct gtgacctgcc tcagactca                                29


<210>   26
<211>   29
<212>   DNA
<213>   Artificial sequence
```

```
<220>
<223>  Primer RvAscImIFNalpha1

<400>  26
gggcgcgcct ttctcttctc tcagtcttc                                        29


<210>  27
<211>  32
<212>  DNA
<213>  Artificial sequence

<220>
<223>  Primer FwAscImApoA1

<400>  27
ccaggcgcgc cggatgaacc ccagtcccaa tg                                    32


<210>  28
<211>  63
<212>  DNA
<213>  Artificial sequence

<220>
<223>  Primer RvmApoA1p17

<400>  28
tcacgcacgc tcataccaag aactcctagg aataaaccaa atacgcttgg gcgcgccctg      60

ggc                                                                    63


<210>  29
<211>  60
<212>  DNA
<213>  Artificial sequence

<220>
<223>  Primer RvmApoA1p144

<400>  29
tcaattctgc atcatggccc agattatcga ggcgtccagc gaggtgggcg cgccctgggc      60


<210>  30
<211>  20
<212>  DNA
<213>  Artificial sequence

<220>
<223>  Primer RvSPmApoA1

<400>  30
ttgctgccat acgtgccaag                                                  20


<210>  31
<211>  66
```

<212> DNA
<213> Artificial sequence

<220>
<223> Primer RvSPmApoA1p17

<400> 31
tcacgcacgc tcataccaag aactcctagg aataaaccaa atacgctttt gctgccagaa    60

atgccg    66


<210> 32
<211> 62
<212> DNA
<213> Artificial sequence

<220>
<223> Primer RvSPmApoA1p144

<400> 32
tcattctgca tcatggccca gattatcgag gcgtccagcg aggtttgctg ccagaaatgc    60

cg    62


<210> 33
<211> 44
<212> DNA
<213> Artificial sequence

<220>
<223> Primer FwMMp9AscIp144

<400> 33
ccaggcgcgc cgcttttccc gacgtctacc tcgctggacg cctc    44


<210> 34
<211> 21
<212> DNA
<213> Artificial sequence

<220>
<223> Primer RvMMp9AscIp144

<400> 34
tcaattctgc atcatggccc a    21


<210> 35
<211> 87
<212> DNA
<213> Artificial sequence

<220>
<223> Primer FwLINKERp144

<400> 35
cgcgccggca ccagcagaaa caaaagcaga accaatgaca acctcgctgg acgcctcgat    60

```
aatctgggcc atgatgcaga attgagc                                                87
```

<210> 36
<211> 87
<212> DNA
<213> Artificial sequence

<220>
<223> Primer RvLINKERp144

<400> 36
```
ggccgctcaa ttctgcatca tggcccagat tatcgaggcg tccagcgagg ttgtcattgg    60

ttctgctttt gtttctgctg gtgccgg                                        87
```

<210> 37
<211> 20
<212> DNA
<213> Artificial sequence

<220>
<223> Primer FwUSP18

<400> 37
```
ccaaaccttg accattcacc                                                20
```

<210> 38
<211> 21
<212> DNA
<213> Artificial sequence

<220>
<223> Primer RvUSP18

<400> 38
```
atgaccaaag tcagcccatc c                                              21
```

<210> 39
<211> 20
<212> DNA
<213> Artificial sequence

<220>
<223> Primer FwISG15

<400> 39
```
gattgcccag aagattggtg                                                20
```

<210> 40
<211> 20
<212> DNA
<213> Artificial sequence

<220>

<223> Primer RvISG15

<400> 40
tctgcgtcag aaagacctca                                          20

<210> 41
<211> 20
<212> DNA
<213> Artificial sequence

<220>
<223> Primer FwIRF1

<400> 41
ccagccgaga cactaagagc                                          20

<210> 42
<211> 20
<212> DNA
<213> Artificial sequence

<220>
<223> Primer RvIRF1

<400> 42
cagagagact gctgctgacg                                          20

<210> 43
<211> 20
<212> DNA
<213> Artificial sequence

<220>
<223> Primer FwMx1

<400> 43
atctgtgcag gcactatgag                                          20

<210> 44
<211> 20
<212> DNA
<213> Artificial sequence

<220>
<223> Primer RvMx1

<400> 44
ctctccttct ttcagcttcc                                          20

<210> 45
<211> 16
<212> DNA
<213> Artificial sequence

<220>

<223> Primer FwmActina

<400> 45
cgcgtccacc cgcgag                                                    16


<210> 46
<211> 16
<212> DNA
<213> Artificial sequence

<220>
<223> Primer RvmActina

<400> 46
cctggtgcct agggcg                                                    16


<210> 47
<211> 19
<212> DNA
<213> Secuencia Artificial

<220>
<223> Primer qPCR FwmIFNa

<400> 47
tctytcytgy ctgaaggac                                                 19


<210> 48
<211> 20
<212> DNA
<213> Artificial sequence

<220>
<223> Primer qPCR RvmIFNa

<400> 48
cacagrggct gtgtttcttc                                                20


<210> 49
<211> 20
<212> DNA
<213> Artificial sequence

<220>
<223> Primer Fw 2-5 OAS

<400> 49
actgtctgaa gcagattgcg                                                20


<210> 50
<211> 20
<212> DNA
<213> Artificial sequence

<220>

```
<223>   Primer Rv 2-5 OAS

<400>   50
tggaactgtt ggaagcagtc                                              20


<210>   51
<211>   15
<212>   DNA
<213>   Artificial sequence

<220>
<223>   Nucleotide sequence encoding the MMP9 protease cleavage site

<400>   51
cttttcccga cgtct                                                   15


<210>   52
<211>   5
<212>   PRT
<213>   Artificial sequence

<220>
<223>   Fibronectin linker fragment

<400>   52

Gly Thr Ser Gly Gln
1               5


<210>   53
<211>   32
<212>   DNA
<213>   Artificial sequence

<220>
<223>   Nucleotide sequence encoding the linker disclosed in SEQ ID NO: 13

<400>   53
gcaccagcag aaacaaaagc agaaccaatg ac                                32


<210>   54
<211>   9
<212>   PRT
<213>   Artificial Sequence

<220>
<223>   péptido AH-1

<400>   54

Ser Pro Ser Tyr Val Tyr His Gln Phe
1               5
```

**Description**

**TECHNICAL FIELD OF THE INVENTION**

The invention is comprised within the field of the methods for stabilizing and targeting compounds of therapeutic interest in a specific manner to target tissues. The invention is particularly based on the capacity of apolipoprotein A to target compounds of therapeutic interest to all those tissues having on their surface binding sites with high affinity for said protein.

**BACKGROUND OF THE INVENTION**

The development of new forms of therapy using macromolecules as active ingredients has generated the need to develop effective forms of stabilizing and targeting said molecules to their suitable cell targets. Examples of therapy requiring the specific targeting to a target tissue include therapies based on the use of specific growth factors or on the use of genes which are used to replace absent or deficient genes in the target tissue. The tissue-specific systems that are not based on viral vectors frequently suffer from the problem of their low or nil cell specificity.

Different systems have been described for targeting therapeutic compounds to liver cells based on lipid vesicles containing the therapeutic compound therein and the targeting of which is given by the presence on the surface of the vesicles of molecules showing affinity for liver cell membranes.

For example, WO07130873 describes methods for targeting microvesicles to liver cells by means of incorporating on the surface of said capsules a compound which is specifically recognized by asialoglycoprotein, hyaluronan, N-acetyl-galactosamine or mannose receptors present in liver cells. WO02086091 describes methods for targeting nanovesicles to liver cells by means of incorporating the hepatitis B virus coat protein in said nanovesicles. WO200473684 describes a method for targeting partially hydrophobic compounds to liver cells based on phospholipid discoidal vesicles comprising ApoA-I on their surface. Lou et al (World J. Gastroenterol., 2005, 11:954-959) have described a method for targeting a lipophilic antitumor compound to the hepatocellular carcinoma cells using high density lipoprotein (HDL) as a specific carrier based on the capacity of HDL to accommodate hydrophobic compounds such as cholesterol.

Finally, Kim et al (Molecular Therapy, 2007, 15:1145-1152) have described a method for targeting interfering RNA to liver cells based on liposomes including interfering RNAs and containing ApoA-I on their surface. However, these methods have the drawback that they only allow carrying hydrophobic compounds since said compounds are housed inside vesicles or artificial membranes in contact with the hydrophobic fraction of the phospholipids.

Alternatively, it is possible to carry hydrophilic compounds to the liver by means of using conjugates of said compounds to agents which are specifically captured by the liver. For example, Kramer et al (J.Biol.Chem., 1992, 267: 18598-18604) have described methods for targeting therapeutic compounds (the cytostatic agent chlorambucil and the prolyl-4 hydroxylase I-nitrobenzo-2-oxa-1,3-diazol-β-Ala-Phe-5-oxaproline-Gly inhibitor) to liver cells by means of the conjugation of said compounds to bile acids. However, this type of conjugation only allows carrying to the liver, which excludes its use for the administration of compounds to other tissues of therapeutic interest.

WO04082720 describes methods for targeting compounds with therapeutic activity to liver cells by means of incorporating said compounds in pseudoviral particles formed by the hepatitis B virus coat protein. However, these vehicles have the problem of showing a reduced plasma half-life which requires a continuous administration or an administration at high doses to reach sustained therapeutic plasma levels. Furthermore, the viral proteins forming the pseudoviral particles generate a humoral immune response.

WO8702061A describes methods for targeting compounds to tissues expressing the LDL receptor by means of using fusion proteins formed by the apoliprotein B or E receptor binding region and an active component.

The problem of the short half-life of the interferon has been dealt with by WO07021494, which describes fusion proteins formed by albumin and interferon. These fusions reach plasma half-lives of about 14 days.

Therefore, there is a need for suitable vehicles for specifically targeting therapeutic compounds to liver cells and which allow reaching a long plasma half-life of the conjugates.

**SUMMARY OF THE INVENTION**

In a first aspect, the invention relates to a conjugate comprising

(i) an Apo A molecule or a functionally equivalent variant thereof and
(ii) a compound of therapeutic interest

wherein components (i) and (ii) are covalently bound.

In a second aspect, the invention relates to a polynucleotide or a gene construct comprising a polynucleotide encoding a conjugate according to the invention wherein the compound of therapeutic interest (ii) is a polypeptide which forms a single chain with component (i).

In successive aspects, the invention relates to a vector comprising a polynucleotide or a gene construct according to the invention and to a host cell comprising a polynucleotide, a gene construct or a vector according to the invention or a nanolipoparticle comprising the conjugate of the invention.

In another aspect, the invention relates to a conjugate, a polynucleotide, a gene construct, a vector, a host cell or a nanolipoparticle according to the invention for its use in medicine.

In another aspect, the invention relates to a conjugate, a polynucleotide, a gene construct, a vector, a host cell or a nanolipoparticle according to the invention for the treatment of liver diseases or of diseases associated with the immune system.

In another aspect, the invention relates to a composition comprising:

(a) a first component selected from the group of a conjugate, a polynucleotide, a gene construct, a vector, a host cell, a nanolipoparticle or a pharmaceutical preparation according to the invention, wherein component (ii) is a TGF-β1 inhibitor peptide and
(b) a second component selected from the group of an immunostimulatory cytokine, a polynucleotide encoding said cytokine, a vector comprising said polynucleotide, a TGF-β1 inhibitory peptide, a cytotoxic agent or a combination thereof.

In another aspect, the invention relates to a combination of the invention for its use in medicine and, in particular, for the treatment of cancer.

**BRIEF DESCRIPTION OF THE DRAWINGS**

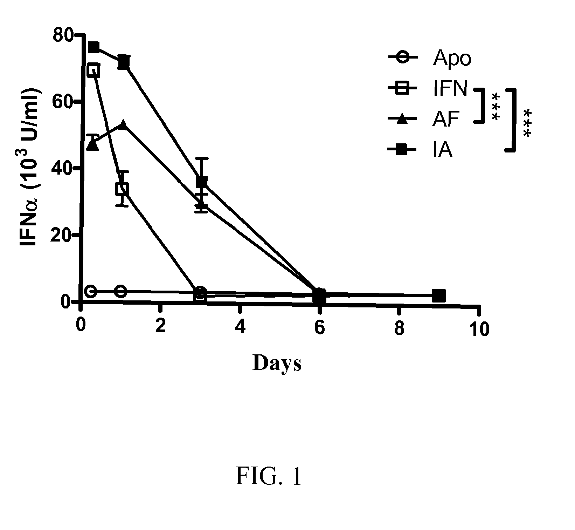

Figure 1. Kinetics of the expression of IFNα. BALB/c mice received a hydrodynamic injection with the plasmids expressing ApoAI (Apo), IFNα (IFN), Apo-IFN (AF) or IFN-Apo (IA). After 6 hours and on day 1, 3, 6 and 9, blood was extracted and the serum IFNα levels were analyzed by means of ELISA. The mean and the standard error of the mean of a representative experiment with four animals per group are shown. The results were analyzed by means of repeated-measures ANOVA, followed by a Bonferroni test. Significant differences were observed between the IFNα levels on day 1 and 3 induced by plasmids AF and IA and the levels induced by plasmid IFN ($p < 0.001$).

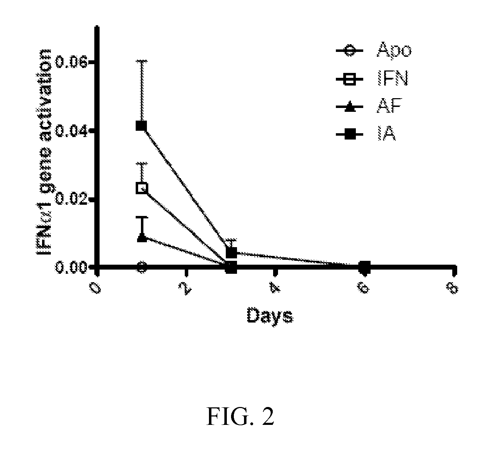

Figure 2. Quantitative RT-PCR of liver mRNA of IFNα1. A hydrodynamic injection was carried out in three BALB/c mice for each plasmid and day of study. On day 1, 3 and 6, the animals were sacrificed and the liver was extracted. The liver mRNA was purified and quantitative RT-PCR was performed for the IFNα1 gene. The mean and the standard error of the mean of a representative experiment are shown. The results were analyzed by means of repeated-measures ANOVA, followed by a Bonferroni test. No significant differences were observed between the mRNA levels induced by plasmids IFNαI, AF and IA.

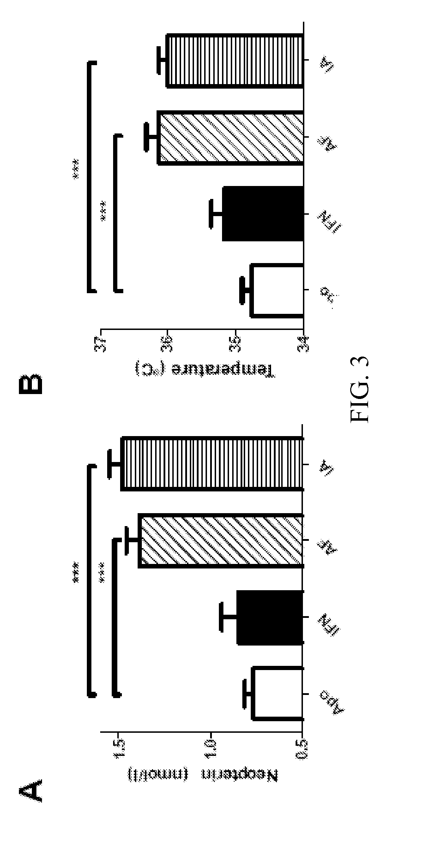

Figure 3. Body temperature and serum neopterin levels. The plasmids encoding the constructs with IFNα were administered to BALB/c mice. On day 3, blood was extracted and the serum neopterin (A) levels were measured by means of ELISA. At the same time, the body temperature (B) was analyzed. The mean and the standard error of the mean of two independent experiments (N=6 mice) are shown. The data was analyzed by means of ANOVA followed by Dunnett's test, comparing the groups with IFNα with the control group with ApoAI. *** $p < 0.0001$.

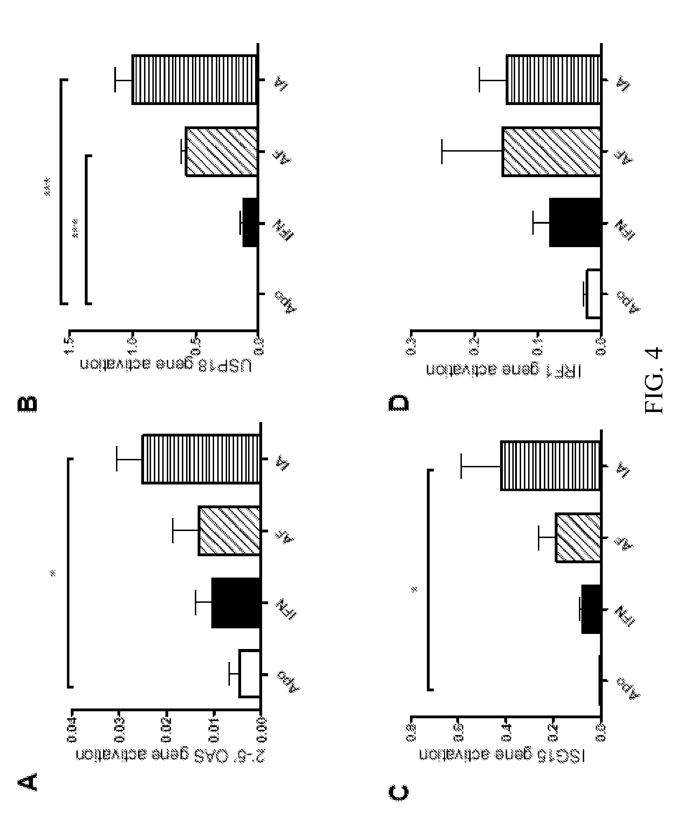

Figure 4. Quantitative RT-PCR of liver mRNA of genes inducible by IFNα1. A hydrodynamic injection was carried out with the plasmids expressing the constructs with IFNα1 in BALB/c mice. On day 3, the animals were sacrificed and the liver was extracted. The liver mRNA was purified and quantitative RT-PCR was performed for the 2'-5' OAS (A), USP18 (B), ISG15 (C) and IRF1 (D) genes. The mean and the standard error of the mean of two independent experiments (N=6 mice) are shown. The data was analyzed by means of ANOVA followed by Dunnett's test, comparing the groups with IFNα with the control group with ApoAI. * $p < 0.05$; *** $p < 0.0001$.

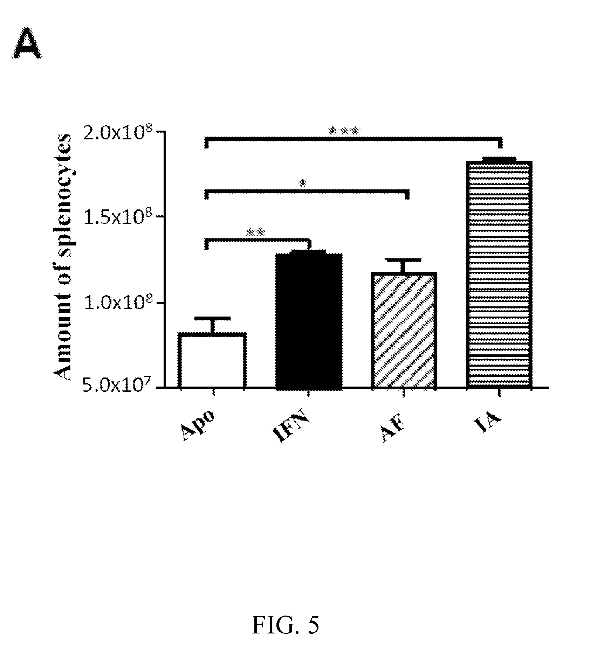

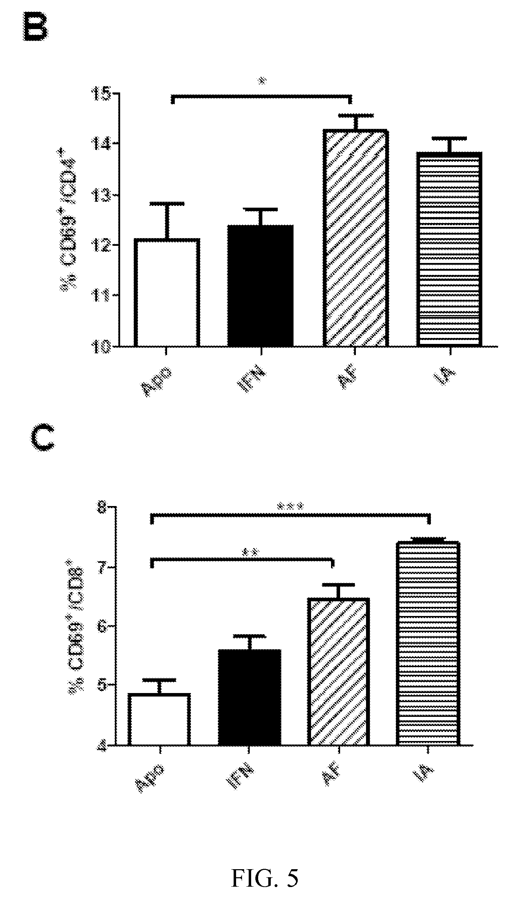

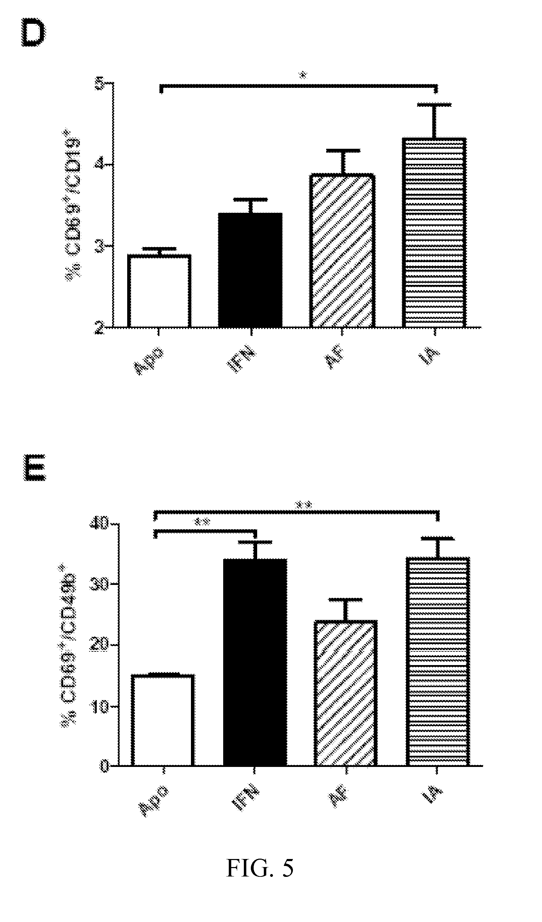

Figure 5. Increase of the number and of the activation of splenocytes. BALB/c mice received the different constructs with IFNα by a hydrodynamic route and six days later, they were sacrificed and the spleens were isolated. The number of splenocytes (A) and the expression of the early activation marker CD69 in CD4+ T cells (B), in CD8+ T cells (C), in B cells (D) and in NK cells (E) were analyzed. The mean and the standard error of the mean of a representative experiment with 7 animals per group are shown.

The data was analyzed by means of ANOVA followed by Dunnett's test, comparing the groups with IFNα with the control group with ApoAI. * p<0.05; ** p<0.001; *** p<0.0001.

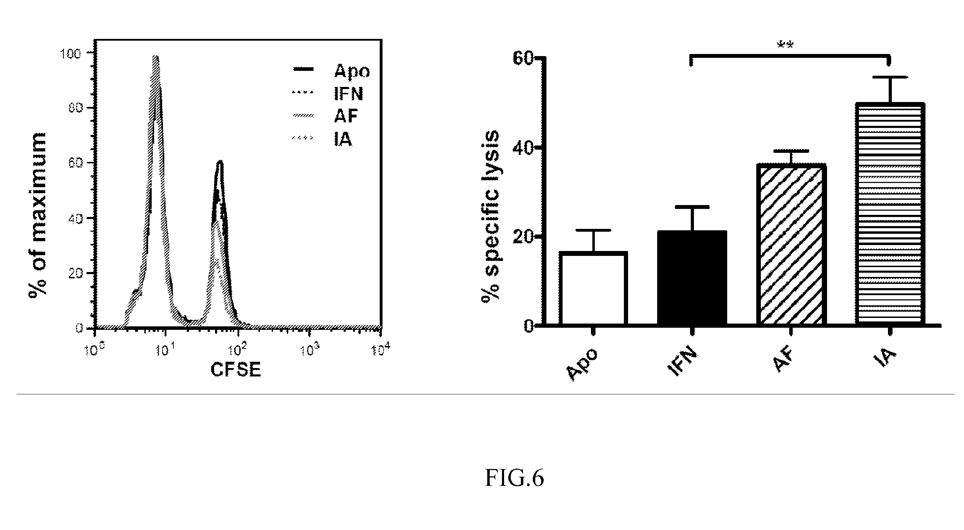

**Figure 6.** Increase of the specific lysis induced by a gene vaccination in the presence of constructs expressing IFNα. BALB/c mice were immunized by means of the hydrodynamic injection of a plasmid expressing β-galactosidase and plasmids expressing the different constructs with IFNα were coadministered as an adjuvant. Seven days later, target cells loaded with a cytotoxic peptide and high concentration of CFSE and control cells with low concentration of CFSE were intravenously injected. After 24 hours, the animals were sacrificed, and the proportion of target cells and control cells was analyzed to calculate the percentage of specific lysis. A histogram representative of each group (A) and the mean and the standard error of the mean of a representative experiment with three animals per group (B) are shown. The data was analyzed by means of ANOVA followed by Dunnett's test, comparing the groups of Apo-IFN and IFN-Apo with the group with IFNα. ** p<0.001.

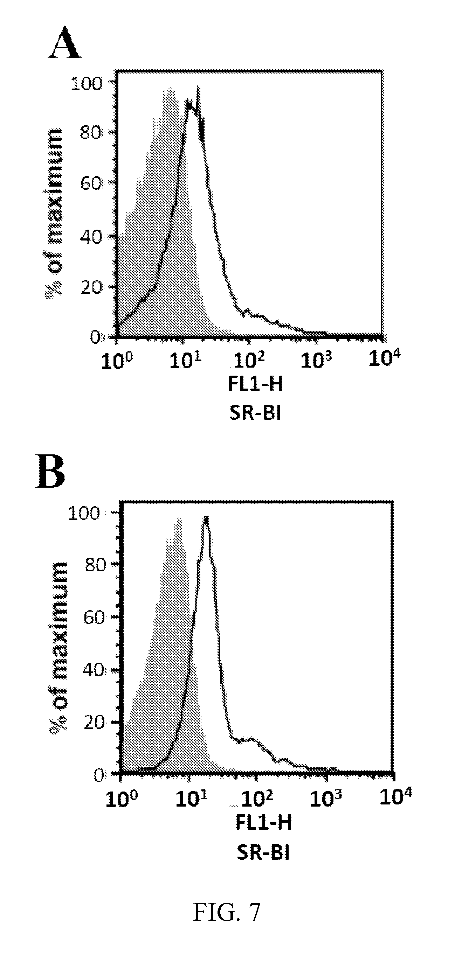

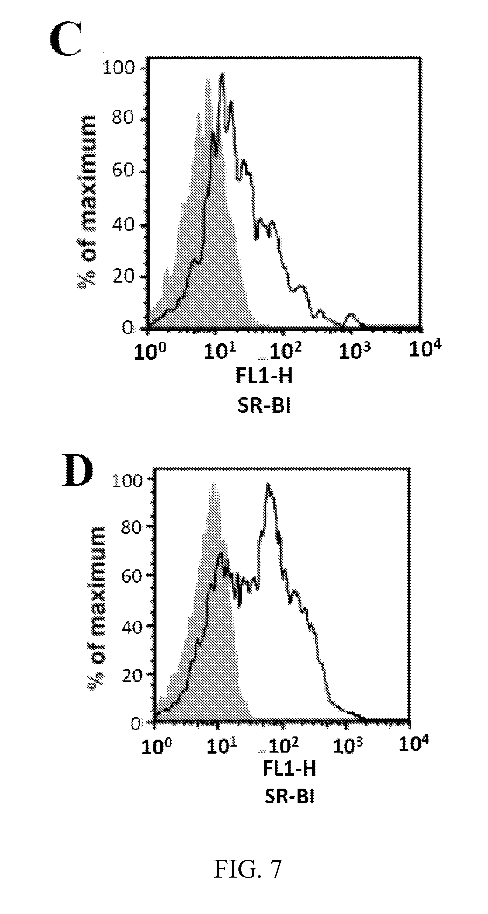

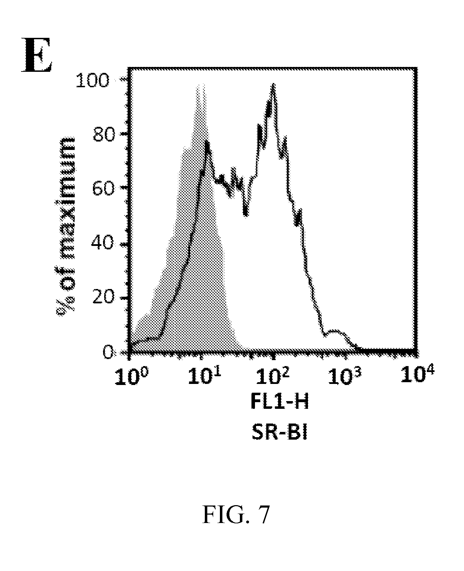

**Figure 7.** Expression of SR-BI in different immune system cell populations. Splenocytes from BALB/c mice spleen were isolated and labeled with anti-SR-BI antibodies and with antibodies to distinguish CD4+ T cells (anti-CD4) (A), CD8+ lymphocytes (anti-CD8) (B), NK cells (anti-CD49b) (C), monocytes/macrophages (anti-CD 11b) (D) or dendritic cells (anti-CD 11c) (E).

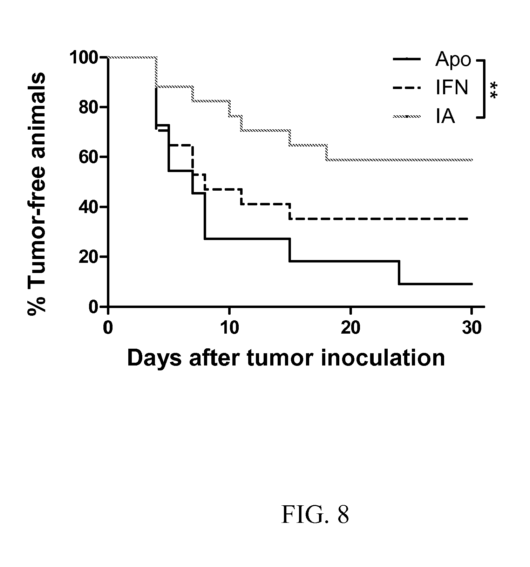

**Figure 8.** Effect of the adjuvant effect in an antitumor vaccination model.
11-17 BALB/c mice for each treatment group received a hydrodynamic injection with the plasmids expressing ApoAI (Apo), IFNα (IFN), or IFN-Apo (IA). 24 hours later, they were vaccinated with the cytotoxic peptide AH1 in Freund's incomplete adjuvant. Nine days later, $5 \times 10^6$ CT26 cells were subcutaneously inoculated and the onset of tumors was observed over time. The percentage of tumor-free mice over time is shown. The experimental groups were compared to the control group by means of the Log-rank test. ** p<0.01.

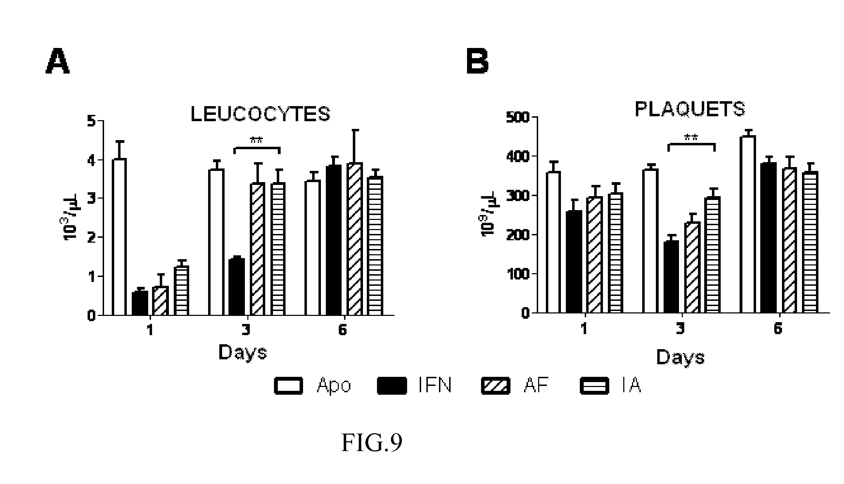

**Figure 9.** Kinetics of circulating leukocytes and platelets. The plasmids encoding the constructs with IFNα were administered to BALB/c mice. Blood was extracted from one group on day 1, from another group on day 3 and from a last group on day 6 after the hydrodynamic injection. The leukocyte (A) and platelet (B) count was quantified using a Z1 Coulter Particle Counter according to the manufacturer's instructions. The mean and the standard error of the mean of two independent experiments (N=4 mice/day and group) are shown. The data was analyzed by means of ANOVA followed by Dunnett's test comparing the groups with IFN-Apo and with the group with IFN. ** p<0.01.

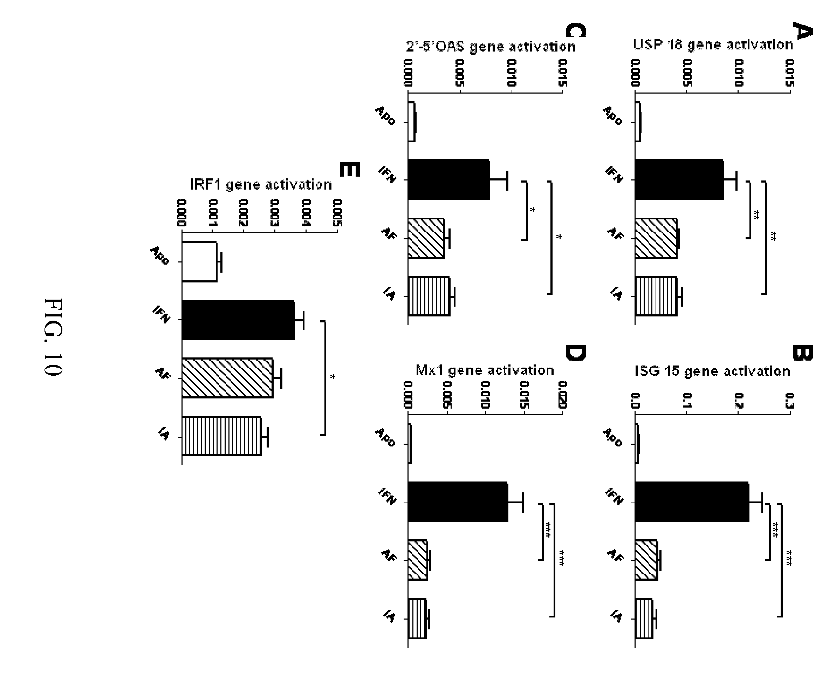

**Figure 10.** Quantitative RT-PCR of brain mRNA of genes inducible by IFNα. A hydrodynamic injection was carried out with the plasmids expressing the constructs with IFNα in BALB/c mice. On day 1, the animals were sacrificed and the brain was extracted. The brain mRNA was purified and quantitative RT-PCR was performed for the USP18 (A), ISG15 (B), 2'-5' OAS (C), Mx1 (D) and IRF1 (D) genes. The mean and the standard error of the mean of two independent experiments (N=5 mice/ group) are shown. The data was analyzed by means of ANOVA followed by Dunnett's test comparing the groups with IFN-Apo with the group with IFN. * p<0.05; ** p<0.01; *** p<0.001. This is one experiment that represents two.

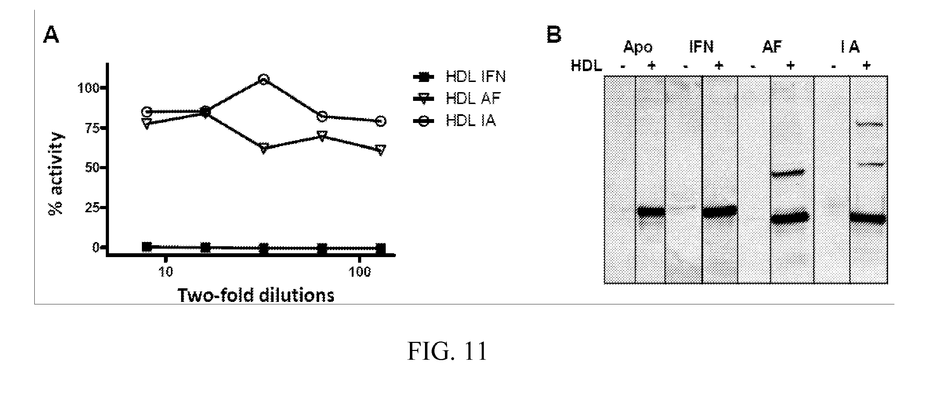

**Figure 11.** Incorporation of the fusion proteins in the circulating high density lipoproteins (HDLs). The plasmids encoding the constructs with IFNα were administered to BALB/c mice. After 24 hours, blood was extracted and from the serum obtained, the HDLs were extracted by means of differential centrifugation in NaBr gradients. The presence of IFNα in the HDLs of the different groups was analyzed by means of an interferon bioassay, the cytopathic effect protection assay (A). With the HDLs-free (HDLs -) serum samples and the fraction containing the HDLs (HDLs +), a western blot was performed to determine the presence of apolipoprotein AI (B).

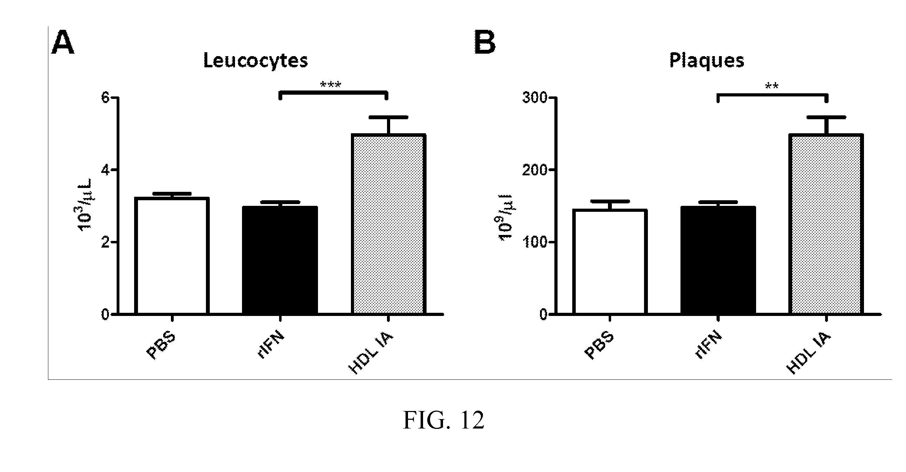

**Figure 12.** Hematological effects of the administration of HDLs containing IFN-Apo. The equivalent to 10000 IU of IFN of HDLs containing IFN-Apo, 10000 IU of recombinant IFN or PBS was administered to BALB/c mice. After 3 days, the leukocyte (A) and platelet (B) count was quantified using a Z1 Coulter Particle Counter according to the manufacturer's instructions. The mean and the standard error of the mean of two independent experiments (N=4-6 mice/group) are shown. The data was analyzed by means of ANOVA followed by Dunnett's test comparing the groups with IFN-Apo and with the group with IFN. ** p<0.01; *** p<0.001.

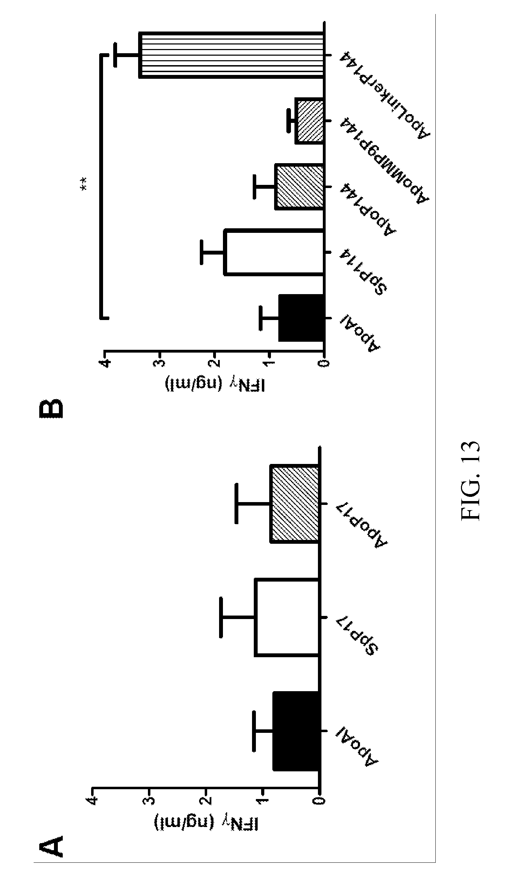

**Figure 13.** Increase of IFNγ induction induced by IL12. A plasmid expressing IL12 under the control of a promoter inducible by doxycycline and another plasmid expressing a control construct or one of the constructs with a TGFβ inhibitor p17 (A) or the TGFβ inhibitor p144 (B) were administered by means of a hydrodynamic injection. After four days, the serum concentration of IFNγ was analyzed by means of ELISA. The mean and the standard error of the mean of a representative experiment with three animals per group are shown. The data was analyzed by means of ANOVA followed by Dunnett's test, comparing the experimental groups with the control group. ** $p < 0.001$.

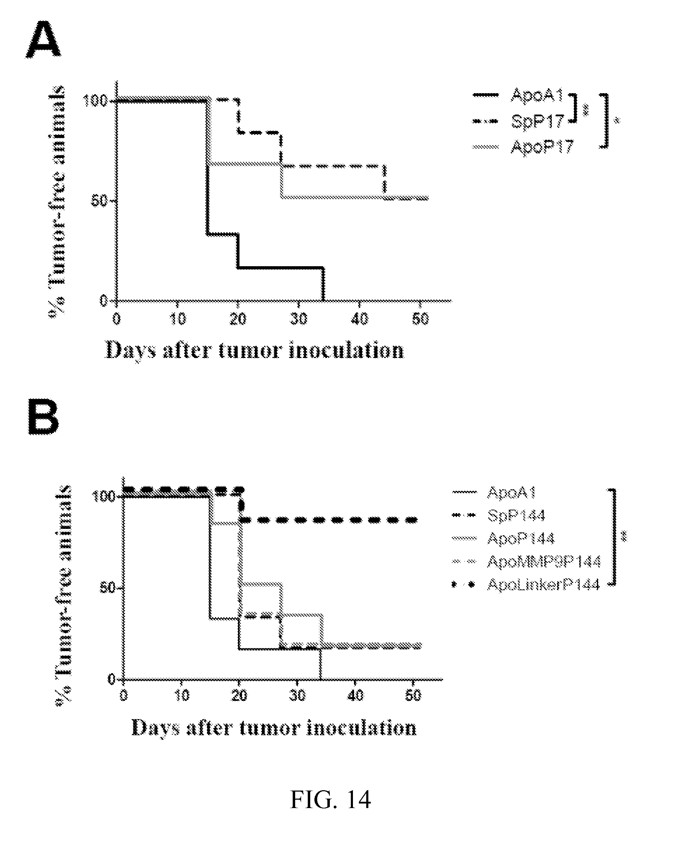

**Figure 14.** Protection against the development of CT26 tumors. BALB/c mice were vaccinated with the cytotoxic peptide AH1 in Freund's incomplete adjuvant. Seven days later, they received a hydrodynamic injection with the constructs expressing TGFβ inhibitors or ApoA-I as a control. After another seven days, $5 \times 10^5$ CT26 cells were subcutaneously inoculated and the onset of tumors was observed over time. The percentage of tumor-free mice over time is shown. The experimental groups were compared to the control group by means of the log-rank test. * $p < 0.05$; ** $p < 0.001$.

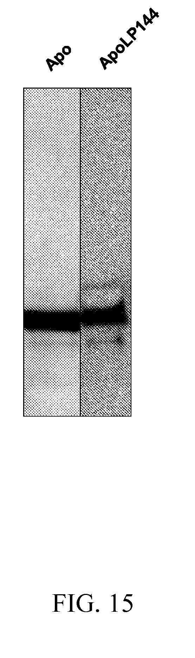

**Figure 15.** Incorporation of the Apo-linker-P144 fusion proteins in the circulating high density lipoproteins (HDLs). The plasmids encoding the constructs with Apo or Apo-linker-P144 were administered to BALB/c mice. After 24 hours, blood was extracted and from the serum obtained, the HDLs were extracted by means of differential centrifugation in NaBr gradients. With the fractions containing the HDLs, a western blot was performed to determine the presence of apolipoprotein AI.

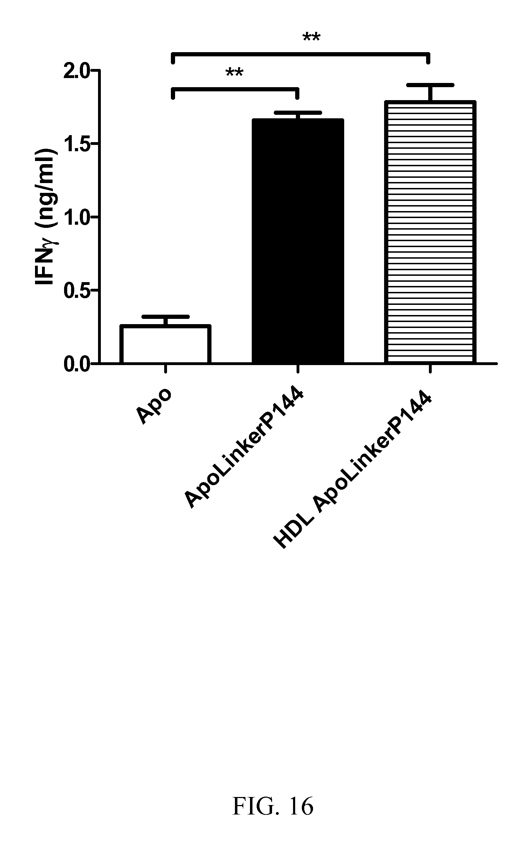

**Figure 16.** Increase of IFNγ induction induced by IL12 after administering HDLs containing Apo-linker-P 144. A plasmid expressing IL12 under the control of a promoter inducible by doxycycline and another plasmid expressing a control construct (Apo) or Apo-linker-P144 were administered by means of a hydrodynamic injection. The plasmid IL12 and an intraperitoneal injection of 14 μg/mouse of HDLs containing Apo-linker-P 144 were administered to a last group. After four days, the serum concentration of IFNγ was analyzed by means of ELISA. The mean and the standard error of the mean of a representative experiment with three animals per group are shown. The data was analyzed by means of ANOVA followed by Dunnett's test comparing the experimental groups with the control group. ** $p < 0.01$.

## DETAILED DESCRIPTION OF THE INVENTION

### 1. Conjugate of the invention

The authors of the present invention have observed that the conjugates formed by an Apo A protein or a functionally equivalent variant thereof and a molecule of therapeutic interest show, after their administration to patients, a serum half-life greater than that observed in patients to whom the molecule of therapeutic interest has been administered without conjugation. Additionally, the conjugates of Apo A and the molecule of therapeutic interest are specifically carried to the liver of the patient, which enormously facilitates the treatment of liver diseases and the reduction of side-effects due to the action of the therapeutic molecule in other tissues.

Thus, in a first aspect, the invention relates to a conjugate comprising

(i) an Apo A molecule or a functionally equivalent variant thereof and
(ii) a compound of therapeutic interest

wherein components (i) and (ii) are covalently bound.

Without intending to be linked to any theory, it is believed that the affinity of the conjugates for liver tissue is due to the fact that said tissue has specific receptors for the Apo A proteins the natural function of which is to capture HDLs having ApoA-I on their surface. In addition, the longer half-life of the conjugates seems to be related to the long half-life that the Apo A molecules show in the organism (in the order of 35 hours in humans or 10 hours in mice in the case of ApoA-I). Furthermore, there are other cells expressing specific receptors for ApoA-I on their surface, which allows the carrying to other tissues.

### 1.1 Apo A Molecule

In the context of the present invention, "Apo A protein" is understood as any member of the Apo A family forming part of the high density lipoproteins (HDLs) and which is capable of interacting specifically with receptors on the surface of liver cells, thus ensuring its capacity to carry the molecules of interest coupled to said Apo A protein to this organ. The Apo A molecules which can be used in the present invention are preferably selected from the group of ApoA-I, ApoA-II, ApoA-III, ApoA-IV and ApoA-V or of functionally equivalent variants thereof.

In a preferred embodiment, the Apo A protein which is used in the present invention is the ApoA-I protein. In the context of the present invention, ApoA-I is understood as the mature form of the pre-proApoA-I protein forming part of the high density lipoproteins (HDLs). ApoA-I is synthesized as a precursor (pre-proApoA-I) containing a secretion signal sequence which is eliminated to give rise to the precursor. The signal sequence is made up of 18 amino acids, the propeptide of 6 amino acids and the mature form of the protein of 243 amino acids. The mature form of the protein which lacks a signal peptide and is processed is preferably used. In a preferred embodiment, the ApoA-I protein is of human origin and its amino acid sequence is that shown in SEQ ID NO:1 (access number in UniProt P02647). In another preferred embodiment, the ApoA-I protein is of murine origin, in particular from mouse, and its amino acid sequence is that shown in SEQ ID NO:2 (access number in UniProt Q00623). In another preferred embodiment, the ApoA-I protein is of murine origin, in particular from rat, and its amino acid sequence is that shown in SEQ ID NO:3 (access number in UniProt P04639).

A functionally equivalent variant of ApoA-I is understood as all those polypeptides resulting from the insertion, substitution or deletion of one or more amino acids of the previously mentioned human or murine ApoA-I sequence and substantially maintaining intact the capacity to interact with the so-called "scavenger receptor class B type I" (SR-BI) forming the HDL receptor present in liver cells. The capacity to interact with the HDL receptor is determined essentially as has been described by Monaco et al (EMBO J., 1987, 6:3253-3260) by means of studies of ApoA-I binding to the hepatocyte membrane or by means of determining the capacity of ApoA-I or of its variant to inhibit the binding of HDL to the hepatocyte membrane receptors. The dissociation constant of the binding of the variant of ApoA-I to the hepatocyte membranes is preferably at least $10^{-8}$ M, $10^{-7}$ M, $10^{-6}$ M, $10^{-5}$ M or $10^{-4}$ M.

Variants of ApoA-I contemplated in the context of the present invention include polypeptides showing at least 60%, 65%, 70%, 72%, 74%, 76%, 78%, 80%, 90% or 95% similarity or identity with the ApoA-I polypeptides. The degree of identity between two polypeptides is determined using computer algorithms and methods that are widely known by persons skilled in the art. The identity between two amino acid sequences is preferably determined using the BLASTP algorithm (BLAST Manual, Altschul, S. et al., NCBI NLM NIH Bethesda, Md. 20894, Altschul, S., et al., J., 1990, Mol. Biol. 215:403-410).

The variants of ApoA-I used in the context of the invention preferably have a long serum half-life with respect to native ApoA-I, which allows reaching serum ApoA-I levels greater than those observed with ApoA-I. Methods for determining the serum half-life of a protein and, in particular of ApoA-I, are known in the art and include, among others, using the methods based on metabolic labeling with labeled proteins described by Eisenberg, S. et al (J.Lipid Res., 1973, 14:446-458), by Blum et al. (J. Clin. Invest., 1977, 60:795-807) and by Graversen et al (J Cardiovasc Pharmacol., 2008, 51:170-177). An example of said variants which shows a longer half-life is, for example, the variant called Milano (which contains the mutation R173C).

*1.2 Compounds of therapeutic interest*

In the context of the present invention, "compounds of therapeutic interest" are understood as any compound which is capable of preventing or eliminating the symptoms of a disease. The invention initially contemplates the use of any therapeutic compound which is susceptible to covalent modification without substantially losing its biological activity, such that it can be conjugated to ApoA-I or to the functionally equivalent variant thereof. Thus, the invention contemplates the use of small organic molecules, peptides, peptidomimetics, peptoids, proteins, polypeptides, glycoproteins, oligosaccharides, nucleic acids and the like as a therapeutically effective component.

By way of example, compounds which can be conjugated to ApoA-I or to the functionally equivalent variant thereof include antibiotics, cholinesterase agents, atropine, scopolamine, sympathomimetic drugs, hypnotic drugs, sedatives, antiepileptic drugs, opioids, analgesics, anti-inflamatory drugs, histamines, lipid derivatives, antiasthmatic drugs, antipyretic-analgesic drugs, xanthines, osmotic diuretics, mercurial compounds, thiazides and sulfonamides, carbonic anhydrase inhibitors, organic nitrates, antihypertensives, cardiac glycosides, antiarrhythmic drugs, oxytocin, prostaglandins, alkaloids, tocolytic agents, antihelminthics, antiprotozoal drugs, antimalarial drugs, amebicides, sulfonamides, penicillins, trimetropin, cephalosporins, sulfamethoxazole, antimycotics, quinolones, antiviral drugs, antibiotics, aminoglycosides, tetracyclines, chloramphenicol, erythromycin, alkylating agents, hormones, antimetabolites, antibiotics, radioactive isotopes, azathioprine, chlorambucil, cyclophosphamide, methotrexate, anticoagulants, thrombolytic drugs, antiplatelet drugs, adenohypophyseal hormones, thyroid and antithyroid hormones, estrogens and progesterone, androgens, adrenocorticotropin, insulin, parathyroid hormone, steroid derivative of vitamin D, vitamins, (hydrosoluble vitamins such as vitamin B complex and ascorbic acid or liposoluble vitamins such as vitamins A, D, K or E), antihistaminic drugs, antitumor, antiviral, antifungal compounds. Compounds which are useful for the treatment of diseases affecting or having their origin in the liver are preferably used.

In a preferred embodiment, component (ii) of the conjugates of the invention comprises a polypeptide chain. In a preferred embodiment, polypeptide ApoA-I and the polypeptide forming component (ii) form a single polypeptide chain. The present invention contemplates the two relative orientations of both polypeptides. Thus, in a preferred embodiment, the C-terminal end of component (i) is bound to the N-terminal end of component (ii). In another preferred embodiment, the N-terminal end of component (i) is bound to the C-terminal end of component (ii). Preferably, when the ApoA-I conjugates are formed by a single polypeptide chain, they are not formed by

(i) the S. *aureus* A protein linked through its C-terminal end to the N-terminal end of the ApoA-I protein.

(ii) the ApoA-I protein linked through its C-terminal end to the N-terminal end of the vasointestinal peptide (VIP-1)

(iii) component (ii) is the immunoglobulin heavy chain of or a plasminogen fragment.

(iv) the tetranectin trimerization domain (TTSE) linked through its C-terminal end to the N-terminal end of the ApoA-I protein.

Polypeptides which can be carried to the liver using the conjugates of the invention include erythropoietin (EPO), leptins, adrenocorticotropin-releasing hormone (CRH), somatotropic hormone-releasing hormone (GHRH), gonadotropin-releasing hormone (GnRH), thyrotropin-releasing hormone (TRH), prolactin-releasing hormone (PRH), melatonin-releasing hormone (MRH), prolactin-inhibiting hormone (PIH), somatostatin, adrenocorticotropin hormone (ACTH), somatotropic hormone or growth hormone (GH), luteinizing hormone (LH), follicle-stimulating hormone (FSH), thyrotropin (TSH or thyroid-stimulating hormone), prolactin, oxytocin, antidiuretic hormone (ADH or vasopressin), melatonin, Müllerian inhibiting factor, calcitonin, parathyroid hormone, gastrin, cholecystokinin (CCK), Arg-vasopressin, thyroid hormones, azoxymethane, triiodothyronine, LIF, amphiregulin, soluble thrombomodulin, SCF, osteogenic protein 1, BMPs, MGF, MGSA, heregulins, melanotropin, secretin, insulin-like growth factor I (IGF-I), insulin-like growth factor II (IGF-II), atrial natriuretic peptide (ANP), human chorionic gonadotropin (hCG), insulin, glucagon, somatostatin, pancreatic polypeptide (PP), leptin, neuropeptide Y, renin, angiotensin I, angiotensin II, factor VIII, factor IX, tissue factor, factor VII, factor X, thrombin, factor V, factor XI, factor XIII, interleukin 1 (IL-1), interleukin 2 (IL-2), interleukin 3 (IL-3), interleukin 4 (IL-4), interleukin 5 (IL-5), interleukin 6 (IL-6), interleukin 7 (IL-7), interleukin 8 (IL-8), interleukin 9 (IL-9), interleukin 10 (IL-10), interleukin 11 (IL-11), interleukin 12 (IL-12), interleukin 13 (IL-13), interleukin 14 (IL-14), interleukin 15 (IL-15) interleukin 16 (IL-16), interleukin 24 (IL-24), tumor necrosis factor alpha (TNF-$\alpha$), interferons alpha, beta, gamma, CD3, CD134, CD137, ICAM-1, LFA-1, LFA-3, chemokines including RANTES 1$\alpha$, MIP-1$\alpha$, MIP-1$\beta$, nerve growth factor (NGF), WT1 protein encoded by the Wilms' tumor suppressor gene, platelet-derived growth factor (PDGF), transforming growth factor beta (TGF-beta), bone morphogenetic proteins (BMPs), fibroblast growth factors (FGF and KGF), epidermal growth factor (EGF and related factors), vascular endothelial growth factor (VEGF), granulocyte colony-stimulating factor (GM-CSF), glial growth factor, keratinocyte growth factor, endothelial growth factor, glial-cell line-derived, neurotrophic factor (GDNF), alpha 1-antitrypsin, tumor necrosis factor, granulocyte-macrophage colony-stimulating factor (GM-CSF), cardiotrophin-1 (CT-1), oncostatin M (OSM), serpin (A1, A2, A3, A4, A5, A6, A7, A8, A9, A10, A11, A12, A13, B1, B2, B3, B4, B5, B6, B7, B8, B9, B10, B11, B12, B13, C1, D1, E1, E2, F1, F2, G1, H1, I1 and I2), cyclosporine, fibrinogen, the EDA domain of fibronectin, lactoferrin, tissue-type plasminogen activator (tPA), chymotrypsin, immunoglobins, hirudin, superoxide dismutase, imiglucerase, $\beta$-Glucocerebrosidase, alglucosidase-$\alpha$, $\alpha$-L-iduronidase, iduronate-2-sulfatase, galsulfase, human $\alpha$-galactosidase A, $\alpha$-1 proteinase inhibitor, lactase, pancreatic enzymes (lipase, amylase, protease), adenosine deaminase, immunoglobulins, albumin, Botulinum toxins type A and B, collagenase, human deoxyribonuclease I, hyaluronidase, papain, L-asparaginase, lepirudin, streptokinase, porphobilinogen deaminase (PBGD), cell transforming factor beta (TGF-$\beta$) inhibitor peptides, IL10 inhibitors, FoxP3 inhibitors, TNF$\alpha$ inhibitors, VEGF inhibitors, PD-1 inhibitors and CD152 inhibitors.

In a preferred embodiment, component (ii) of the conjugate of the invention is an interferon (IFN). Interferons are classified as interferon of type I, of type II and of type III. Type I interferons are a polypeptide family with cytokine activity which were originally discovered as a result their inhibitory activity on the viral infection of cell lines *in vitro* (Pestka, S., Krause, C.D. and Walter, M.R. 2004. Immunol Rev. 202:8-32) and which are characterized in that they are bound to the so-called IFN-$\alpha$ receptor (IFNAR). Depending on the homology of their sequences, type I interferons are classified as interferon-$\alpha$ (IFN-$\alpha$), interferon-$\beta$ (IFN-$\beta$) and interferon (IFN-$\omega$). IFN-$\alpha$ and IFN-$\beta$ share a single dimeric receptor which is expressed on the surface of most nucleated cells. The function of these cytokines is very important in the immune response against multiple types of viral infections, given that they start up mechanisms promoting the death by apoptosis of the infected cells and viral replication inhibition while at the same time they favor antigen presentation. It has recently been experimentally documented that it also carries out its functions by directly activating the activities of T, B and NK cells as well as of dendritic cells in the immune response (Le Bon A. et al., 2003. Nat. Immunol. 4: 1009-1015; Le Bon A. et al., 2006. J. Immunol. 176:4682-4689; Le Bon A. et al., 2006. J Immunol. 176:2074-8). Type II interferons are characterized in that they are bound to the interferon gamma receptor (IFNGR) and include IFN-$\gamma$ as a single member. Type III interferons transduce their signal through a complex formed by the IL-10 receptor 2 (IL10R2) and the IFN lambda 1 receptor (IFNLR1) and is formed by three interferons lambda called IFN-$\lambda_1$, IFN-$\lambda_2$ and IFN-$\lambda$3.

In a preferred embodiment, component (ii) is a type I interferon, such as IFN-$\alpha$, IFN-$\beta$, IFN-$\delta$, IFN-$\varepsilon$, IFN-$\kappa$, IFN-$\tau$ and IFN-$\omega$. In a particular embodiment, at least one type I interferon comprised in the composition of the invention is selected from the group comprising interferon-alpha (IFN-$\alpha$) and interferon-beta (IFN-$\beta$). When the type I interferon is IFN-$\alpha$, the latter can correspond to any interferon encoded by any gene member of the family of human IFN-$\alpha$ genes. In a particular embodiment, at least one type I interferon is an IFN-$\alpha$ selected from the group of IFN-$\alpha$2a, IFN-$\alpha$2b, IFN-$\alpha$4, IFN-$\alpha$5, IFN-$\alpha$8 and combinations thereof, including its combination with other substances in pharmaceutical formulations. In an even more particular embodiment, the interferon is IFN-$\alpha$1, preferably of human origin. In a preferred embodiment, the interferon is IFN-$\alpha$5.

A list of species of type I interferon, particularly IFN-$\alpha$ and IFN-$\beta$ which can be used according to the invention, can be found in Bekisz et al. (Growth Factors, 2004; 22: 243-251) and in Petska et al. (Immunological Reviews, 2004; 202: 8-32). Additionally, the invention provides the use of combinations of conjugates comprising more than one type of interferon, such as for example IFN-$\alpha$n1 (lymphoblastoid derivative) or IFN-$\alpha$3 (combination of interferons produced by human leukocytes stimulated with the Sendai virus (or another virus) or viral particles).

The origin of the type I interferon used is not a critical aspect of the invention. This can be of natural origin, extracted and purified from biological fluids or tissues, or produced by means of conventional recombinant genetic engineering and methods, such as those described in Sambrook and Russel ("Molecular Cloning: to Laboratory manual" of J. Sambrook, D.W. Russel Eds. 2001, third edition, Cold Spring Harbor, New York), by synthesis processes or by any other conventional technique described in the state of the art.

In a particular embodiment of the invention, at least one type I interferon comprised in the composition of the invention is in pegylated form. Some examples for preparing pegylated forms of interferon can be found in US5762923 and US5766582. In addition, it is also possible to use some of the interferon forms which are already commercially available, either pegylated or non-pegylated forms. These include, without involving any limitation, ROFERON-A (human recombinant IFN-$\alpha$2a) and PEGASYS (pegylated IFN-$\alpha$) from Hoffmann La Roche Inc., INTRON-A (human recombinant IFN-$\alpha$2b) and PEG-INTRON (pegylated IFN-$\alpha$2b) from Schering Corp., ALFERON-N (IFN-$\alpha$3n, combination of interferons of natural origin) from Interferon Sciences, or IFNERGEN (IFN-$\alpha$con1) from InterMune Pharmaceuticals Inc., the sequence of which is a consensus sequence that does not exactly correspond with a natural sequence. IFN-$\beta$ formulations, such as for example AVONEX (IFN-$\beta$1a) from Biogen Idec, REBIF (IFN-$\beta$1a) from EMD Serono, Inc, and BETASERON (IFN-$\beta$1b) from Bayer Health Care are also included.

In a preferred embodiment, the conjugate of the invention is formed by ApoA-I fused through its C-terminal end and by means of a flexible linker with the N-terminal end of an interferon $\alpha$1 molecule. In another preferred embodiment, the conjugate of the invention is formed by an interferon $\alpha$1 molecule fused through its C-terminal end and by means of a flexible linker with the N-terminal end of a ApoA-I molecule.

In another preferred embodiment, component (ii) is a TGF-beta inhibitor. TGF-beta inhibitors which can form part of the conjugates according to the invention include the peptide inhibitors selected from TGF-beta1 receptor sequences which are bound to the receptor binding site in TGF-$\beta$1, thus blocking the binding to the receptor. These types of peptides have been described in W0200031135, the entire content of which is incorporated by reference. In a preferred embodiment, the TGF-$\beta$1 inhibitor peptide is derived from the TGF-$\beta$1 type III receptor. In an even more preferred embodiment, the inhibitor peptide is peptide p144 having the sequence TSLDASIIWAMMQN (SEQ ID NO:4).

The invention likewise provides the use of inhibitor peptides inhibiting the interaction between TGF$\beta$1 and the TGF$\beta$1 receptor and the signaling occurring in response to said interaction, identified as phage-displayed gene libraries as they have been described in WO200519244, the entire content of which is incorporated by reference. In a preferred embodiment, the inhibitor peptide is peptide p17 characterized by the sequence KRIWFIPRSSWYERA (SEQ ID NO: 5), as well as truncated variants thereof and which substantially conserve the capacity to inhibit the interaction between TGF$\beta$1 and its receptor as they have been described in WO2007048857, the entire content of which is incorporated in the present invention.

*1.3. Linker element between component ApoA and the therapeutically active compound*

The conjugates object of the invention comprising the Apo A protein and a second component with a peptide nature can contain a bond directly connecting the Apo A protein and said second component or, alternatively, can contain an additional amino acid sequence acting as a linker between the Apo A protein and said second component with a peptide nature. According to the invention, said non-natural intermediate amino acid sequence acts as a hinge region between domains, allowing them to move independently from one another while they maintain the three-dimensional shape of the individual domains. In this sense, a preferred non-natural intermediate amino acid sequence according to the invention would be a hinge region characterized by a structural ductility allowing this movement. In a particular embodiment, said non-natural intermediate amino acid sequence is a non-natural flexible linker. In a preferred embodiment, said flexible linker is a flexible linker peptide with a length of 20 amino acids or less. In a more preferred embodiment, the linker peptide comprises 2 amino acids or more selected from the group consisting of glycine, serine, alanine and threonine. In a preferred embodiment of the invention, said flexible linker is a polyglycine linker. Possible examples of linker/spacer sequences include SGGTSGSTSGTGST (SEQ ID NO:6), AGSSTGSSTGPGSTT (SEQ ID NO:7) or GGSGGAP (SEQ ID NO:8). These sequences have been used for binding designed coiled helixes to other protein domains (Muller, K.M., Arndt, K.M. and Alber, T., Meth. Enzymology, 2000, 328: 261-281). Said linker preferably comprises or consists of the amino acid sequence GGGVEGGG (SEQ ID NO:9).

The effect of the linker region is providing space between the Apo A protein and component (ii). It is thus ensured that the secondary structure of Apo A is not affected by the presence of component (ii) and vice versa. The spacer preferably has a peptide nature. The linker peptide preferably comprises at least two amino acids, at least three amino acids, at least five amino acids, at least ten amino acids, at least 15 amino acids, at least 20 amino acids, at least 30 amino acids, at least 40 amino acids, at least 50 amino acids, at least 60 amino acids, at least 70 amino acids, at least 80 amino acids, at least 90 amino acids or approximately 100 amino acids.

The linker can be bound to components flanking the two components of the conjugates of the invention by means of covalent bonds and preferably the spacer is essentially non-immunogenic and/or does not comprise any cysteine residue. In a similar manner, the three-dimensional structure of the spacer is preferably linear or substantially linear.

Preferred examples of spacer or linker peptides include those which have been used for binding proteins without substantially deteriorating the function of the bound proteins or at least without substantially deteriorating the function of one of the bound proteins. More preferably, the spacers or linkers have been used for binding proteins comprising structures with coiled helixes.

The linker can include residues 53-56 of tetranectin, forming a β sheet in tetranectin, and residues 57-59 forming a turn in the tetranectin (Nielsen, B.B. et al., FEBS Lett. 412: 388-396, 1997). The sequence of the segment is GTKVHMK (SEQ ID NO:10). This linker has the advantage that when it is present in native tetranectin, it binds the trimerization domain with the CRD domain, and therefore it is suitable for connecting the trimerization domain to another domain in general. Furthermore, the resulting construct is not expected to be more immunogenic than the construct without a linker.

Alternatively, a subsequence from the connecting strand 3 from human fibronectin can be chosen as a linker, corresponding to amino acids 1992-2102 (SWISSPROT numbering, entry P02751). The subsequence PGTSGQQPSVGQQ (SEQ ID NO:11) corresponding to amino acids number 2037-2049 is preferably used, and within that subsequence fragment GTSGQ (SEQ ID NO:52) corresponding to amino acids 2038-2042 is more preferable. This construct has the advantage that it not very prone to proteolytic cleavage and is not very immunogenic because fibronectin is present at high concentrations in plasma.

Alternatively, a suitable peptide linker can be based on the 10 amino acid residue sequence of the upper hinge region of murine IgG3. This peptide (PKPSTPPGSS, SEQ ID NO: 12) has been used to produce antibodies dimerized by means of a coiled helix (Pack P. and Pluckthun, A., 1992, Biochemistry 31:1579-1584) and can be useful as a spacer peptide according to the present invention. A corresponding sequence of the upper hinge region of human IgG3 can be even more preferable. Human IgG3 sequences are not expected to be immunogenic in human beings.

In a preferred embodiment, the linker peptide is selected from the group of the peptide of sequence APAETKAEPMT (SEQ ID NO:13) and of the peptide of sequence GAP.

Alternatively, the two components of the conjugates of the invention can be connected by a peptide the sequence of which contains a cleavage target for a protease, thus allowing the separation of ApoA-I from component (ii). Protease cleavage sites suitable for their incorporation into the polypeptides of the invention include enterokinase (cleavage site DDDDK, SEQ ID NO:14), factor Xa (cleavage site IEDGR, SEQ ID NO:15), thrombin (cleavage site LVPRGS, SEQ ID NO:16), TEV protease (cleavage site ENLYFQG, SEQ ID NO:17), PreScission protease (cleavage site LEVLFQGP, SEQ ID NO:18), inteins and the like. In a preferred embodiment, the cleavage site is a protease cleavage site expressed in tumor tissues, in inflamed tissues or in liver such that the separation of Apo A and of component (ii) takes place once the conjugate has reached the liver. In a preferred embodiment, the linker contains a matrix metalloprotease-9 recognition site (cleavage site LFPTS, SEQ ID NO:19).

## 2. Methods for obtaining the conjugates of the invention

The conjugates of the invention can be obtained using any method known for a person skilled in the art. It is thus possible to obtain the ApoA protein or the variant of said protein by any standard method. For example, the ApoA-I protein can be purified from serum samples of individuals or of laboratory animals (W09807751, W09811140, Jackson et al., 1976, Biochim Biophys Acta. 420:342-349, Borresen, A.L. and Kindt, T.J., 1978, J. Immunogenet. 5:5-12 and Forgez, P, and Chapman, M.J., 1982, J. Biochem. Biophys. Methods, 6:283-96). Alternatively, the ApoA-I protein can be obtained from cDNA by means of expression in a heterologous organism such as, for example, *E.coli, S.cerevisiae, P.pastoris,* insect cells using methods known in the art such as those described in WO07023476, WO9525786, WO8702062, Feng et al., (Protein. Expr. Purif., 2006, 46:337-42), Pyle et al., 1996 Biochemistry. 35:12046-52), Brissette et al., (Protein Expr. Purif. 1991, 2:296-303) and Bonen, D.K. (J. Biol. Chem., 1997, 272:5659-67).

Once there is a sufficient amount of purified ApoA protein, it must be conjugated to the therapeutic compound of interest. The conjugation of therapeutically active component (ii) to the Apo A molecule can be carried out in different ways. One possibility is the direct conjugation of a functional group to the therapeutically active component in a position which does not interfere with the activity of said component. As understood in the present invention, functional groups relates to a group of specific atoms in a molecule which are responsible for a characteristic chemical reaction of said molecule. Examples of functional groups include, but are not limited to hydroxy, aldehyde, alkyl, alkenyl, alkynyl, amide, carboxamide, primary, secondary, tertiary and quaternary amines, aminoxy, azide, azo (diimide), benzyl, carbonate, ester, ether, glyoxylyl, haloalkyl, haloformyl, imine, imide, ketone, maleimide, isocyanide, isocyanate, carbonyl, nitrate, nitrite, nitro, nitroso, peroxide, phenyl, phosphine, phosphate, phosphono, pyridyl, sulfide, sulfonyl, sulfinyl, thioester, thiol and oxidized 3,4-dihydroxyphenylalanine (DOPA) groups. Examples of said groups are maleimide or glyoxylyl groups which react specifically with thiol groups in the Apo A molecule and oxidized 3,4-dihydroxyphenylalanine (DOPA) groups which react with primary amine groups in the Apo A molecule.

Another possibility is to conjugate therapeutically active component (ii) to the Apo A molecule by means of the use of homo- or heterobifunctional groups. The bifunctional group can be conjugated first to the therapeutically active compound and, then, conjugated to the Apo A protein or, alternatively, it is possible to conjugate the bifunctional group to the Apo A protein and then, conjugate it to the therapeutically active compound. Illustrative examples of theses types of conjugates include the conjugates known as ketone-oxime (described in US20050255042) in which the first component of the conjugate comprises an aminoxy group which is bound to a ketone group present in a heterobifunctional group which is in turn bound to an amino group in the second component of the conjugate.

In other embodiments, the agent which is used to conjugate components (i) and (ii) of the conjugates of the invention can be photolytically, chemically, thermally or enzymatically processed. It is particularly interesting to use linking agents which can be hydrolyzed by enzymes which are in the cell target, so that the therapeutically active compound is only released in the inside of the cell. Examples of types linking agents which can be intracellularly processed have been described in WO04054622, WO06107617, WO07046893 and W007112193.

In a preferred embodiment, component (ii) of the conjugate of the invention is a compound with a peptide nature, including both oligopeptides and peptides. Methods for chemically modifying a polypeptide chain are widely known for a person skilled in the art and include methods based on the conjugation through the thiol groups present in the cysteine moieties, methods based on the conjugation through the primary amino groups present in lysine moieties (US6809186), methods based on the conjugation through the N- and C-terminal moieties. Reagents suitable for modifying polypeptides to allow their coupling to other compounds include: glutaraldehyde (it allows binding compounds to the N-terminal end of polypeptides), carbodiimide (it allows binding the compound to the C-terminal end of a polypeptide), succinimide esters (for example MBS, SMCC) which allow activating the N-terminal end and cysteine moieties, benzidine (BDB), which allows activating tyrosine moieties, periodate, which allows activating carbohydrate moieties in the proteins which are glycosylated.

In the particular case in which component ApoA and the therapeutic compound of interest form a single peptide chain, it is possible to express the conjugate in a single step using a gene construct of the invention encoding said conjugate, for which said construct is introduced in a vector suitable for its expression in a heterologous organism together with transcription and, optionally, translation control elements. The transcription and, optionally, translation control elements present in the expression cassette of the invention include promoters, which direct the transcription of the nucleotide sequence to which they are operatively linked and other sequences which are necessary or suitable for the transcription and its suitable regulation in time and place, for example, initiation and termination signals, cleavage sites, polyadenylation signal, replication origin, transcriptional enhancers, transcriptional silencers, etc. Said elements, as well as the vectors used for constructing the expression cassettes and the recombinant vectors according to the invention are generally chosen according to the host cells to be used.

3. Polynucleotides, gene constructs, vectors and host cells of the invention.

In another aspect, the invention relates to a polynucleotide encoding a polypeptide of the invention. A person skilled in the art will understand that the polynucleotides of the invention will only encode the conjugates in which component (ii) has a peptide nature and in which the polypeptide Apo A forms a single peptide chain, regardless of the relative orientation and regardless of the fact that both components are directly connected or separated by a spacer region.

In another aspect, the invention relates to a gene construct comprising a polynucleotide of the invention. The construct preferably comprises the polynucleotide of the invention located under the operative control of sequences regulating the expression of the polynucleotide of the invention. A person skilled in the art will understand that the polynucleotides of the invention must access the nucleus of a target tissue and there be transcribed and translated to give rise to the biologically active fusion protein. For this reason, when the active ingredient which is administered is a polynucleotide, the latter must preferably encode the precursor form pre-proApoA1 or the precursor form of the ApoA1 variant, such that after its expression it is secreted as a result of the signal sequence and it is processed to give rise to the mature ApoA1.

In the event that the conjugate formed by Apo A fused through its C-terminal end with an interferon molecule is to be expressed, it is preferable for the polynucleotide encoding it to be preceded by a sequence encoding the ApoA1 signal sequence. In the event that the conjugate formed by an interferon molecule fused through its C-terminal end with the N-terminal end of a ApoA molecule is to be expressed, it is preferable for the polynucleotide encoding it to be preceded by a sequence encoding the interferon $\alpha 1$ signal sequence.

In principle, any promoter can be used for the gene constructs of the present invention provided that said promoter is compatible with the cells in which the polynucleotide is to be expressed. Thus, promoters suitable for the embodiment of the present invention include, without being necessarily limited to, constitutive promoters such as the derivatives of the genomes of eukaryotic viruses such as the polyoma virus, adenovirus, SV40, CMV, avian sarcoma virus, hepatitis B virus, the promoter of the metallothionein gene, the promoter of the herpes simplex virus thymidine kinase gene, retrovirus LTR regions, the promoter of the immunoglobulin gene, the promoter of the actin gene, the promoter of the EF-1alpha gene as well as inducible promoters in which the expression of the protein depends on the addition of a molecule or an exogenous signal, such as the tetracycline system, the NFκB/UV light system, the Cre/Lox system and the promoter of heat shock genes, the regulatable promoters of RNA polymerase II described in WO/ 2006/135436 as well as tissue-specific promoters. In a preferred embodiment, the gene constructs of the invention contain the expression-enhancing regions present in promoter regions of predominantly hepatic expression genes such as human serum albumin genes, prothrombin genes, the alpha-1-microglobulin genes or aldolase genes, either in a single copy in the form of several copies thereof and either in an isolated form or in combination with other liver-specific expression elements such as cytomegalovirus, alpha-1-antitrypsin or albumin promoters.

Other examples of promoters which are tissue-specific include the promoter of the albumin gene (Miyatake et al., 1997, J. Virol, 71:5124-32), the core promoter of hepatitis virus (Sandig et al, 1996, Gene Ther., 3:1002-9); the promoter of the alpha-phetoprotein gene (Arbuthnot et al., 1996, Hum.GeneTher., 7:1503-14), and the promoter of the globulin-binding protein which binds to thyroxine (Wang, L., et al., 1997, Proc. Natl. Acad. Sci. USA 94:11563-11566).

The polynucleotides of the invention or the gene constructs forming them can form part of a vector. Thus, in another aspect, the invention relates to a vector comprising a polynucleotide or a gene construct of the invention. A person skilled in the art will understand that there is no limitation as regards the type of vector which can be used because said vector can be a cloning vector suitable for propagation and for obtaining the polynucleotides or suitable gene constructs or expression vectors in different heterologous organisms suitable for purifying the conjugates. Thus, suitable vectors according to the present invention include expression vectors in prokaryotes such as pUC18, pUC19, Bluescript and their derivatives, mp18, mp19, pBR322, pMB9, ColE1, pCR1, RP4, phages and shuttle vectors such as pSA3 and pAT28, expression vectors in yeasts such as vectors of the type of 2 micron plasmids, integration plasmids, YEP vectors, centromeric plasmids and the like, expression vectors in insect cells such as the pAC series and pVL series vectors, expression vectors in plants such as vectors of expression in plants such as pIBI, pEarleyGate, pAVA, pCAMBIA, pGSA, pGWB, pMDC, pMY, pORE series vectors and the like and expression vectors in superior eukaryotic cells based on viral vectors (adenoviruses, viruses associated to adenoviruses as well as retroviruses and lentiviruses) as well as non-viral vectors such as pSilencer 4.1-CMV (Ambion), pcDNA3, pcDNA3.1/hyg pHCMV/Zeo, pCR3.1, pEF1/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

The vector of the invention can be used to transform, transfect or infect cells which can be transformed, transfected or infected by said vector. Said cells can be prokaryotic or eukaryotic. By way of example, the vector wherein said DNA sequence is introduced can be a plasmid or a vector which, when it is introduced in a host cell, is integrated in the genome of said cell and replicates together with the chromosome (or chromosomes) in which it has been integrated. Said vector can be obtained by conventional methods known by the persons skilled in the art (Sambrok et al., 2001, mentioned above).

Therefore, in another aspect, the invention relates to a cell comprising a polynucleotide, a gene construct or a vector of the invention, for which said cell has been able to be transformed, transfected or infected with a construct or vector provided by this invention. The transformed, transfected or infected cells can be obtained by conventional methods known by persons skilled in the art (Sambrok et al., 2001, mentioned above). In a particular embodiment, said host cell is an animal cell transfected or infected with a suitable vector.

Host cells suitable for the expression of the conjugates of the invention include, without being limited to, mammal, plant, insect, fungal and bacterial cells. Bacterial cells include, without being limited to, Gram-positive bacterial cells such as species of the Bacillus, Streptomyces and Staphylococcus genus and Gram-negative bacterial cells such as cells of the Escherichia and Pseudomonas genus. Fungal cells preferably include cells of yeasts such as *Saccharomyces, Pichia pastoris* and *Hansenula polymorpha*. Insect cells include, without being limited to, Drosophila cells and Sf9 cells. Plant cells include, among others, cells of crop plants such as cereals, medicinal, ornamental or bulbous plants. Suitable mammal cells in the present invention include epithelial cell lines (porcine, etc.), osteosarcoma cell lines (human, etc.), neuroblastoma cell lines (human, etc.), epithelial carcinomas (human, etc.), glial cells (murine, etc.), hepatic cell lines (from monkey, etc.), CHO (Chinese Hamster Ovary) cells, COS cells, BHK cells, HeLa cells, 911, AT1080, A549, 293 or PER.C6, NTERA-2 human ECC cells, D3 cells of the mESC line, human embryonic stem cells such as HS293 and BGV01, SHEF1, SHEF2 and HS181, NIH3T3 cells, 293T, REH and MCF-7 and hMSC cells.

In another aspect, the invention relates to a nanolipopartícle that comprises a conjugate according to the invention.

As used herein, the terrm "nanolipoparticle" is equivalent to the terms "lipoprotein" or "lipoprotein particle" and can be used interchangeably. By "nanolipoparticle" is understood herein any hidrosoluble particle, formed by a core of apolar lipids (such as esterified cholesterol and triglycerides) coated by an external polar coat formed by apolipoprtoeins, phospholipids and free cholesterol.

The nanolipoparticles or liporpteins are classified according to their density as chylomicrons, very low density lipoproteins (VLDL), intermediate density lipoproteins (IDL), low density lipoproteins (LDL) and high density lipoproteins (HDL). The features of th different lipoproteins is shown in Table 1.

Table 1

| Density (g/mL) | Class | Diameter (nm) | % protein | % cholesterol | % phospholipid | % triacylglycerol |
|---|---|---|---|---|---|---|
| >1.063 | HDL | 5-15 | 33 | 30 | 29 | 8 |
| 1.019-1.063 | LDL | 18-28 | 25 | 50 | 21 | 4 |
| 1.006-1.019 | IDL | 25-50 | 18 | 29 | 22 | 31 |
| 0.95-1.006 | VLDL | 30-80 | 10 | 22 | 18 | 50 |
| <0.95 | chylomicrons | 100-1000 | <2 | 8 | 7 | 84 |

In a particular, embodiment, the nanolipoparticles according to the invention is an HDL which composition is given in Table 1 and wherein the protein fraction is formed by Apo A, Apo C, Apo D and Apo E.

The nanoparticules of the invention may be obtained using methods known to a skilled artisan. By way of example, the nanolipoparticles may be obtained *in vitro* by the addition of cholesterol and phosphatidylcholine to the conjugate of the invention as described by Lerch, et al. (Vox Sang, 1996, 71: 155-164) or in vivo by the use of a transgenic non-human animal which expresses in the liver the conjugate of the invention, resulting in the secretion to the serum of nanoparticles from where they can be isolated.

4. Medical uses of the conjugates of the invention

The conjugates of the invention are useful for carrying compounds of therapeutic interest to the liver and stabilizing them. Therefore, in another aspect, the invention relates to a pharmaceutical preparation comprising a therapeutically effective amount of a conjugate, of a polynucleotide, of a gene construct, of a vector, or a host cell or of a nanolipoparticle according to the invention and a pharmaceutically acceptable carrier or excipient.

In another aspect, the invention relates to a polypeptide of the invention, a polynucleotide of the invention, a gene construct of the invention, a vector of the invention, a nanolipoparticle of the invention or a pharmaceutical composition for its use in medicine.

For the use in medicine, the conjugates of the invention can be found in the form of prodrug, salt, solvate or clathrate, either in an isolated form or in combination with additional active agents. The combinations of compounds according to the present invention can be formulated together with an excipient which is acceptable from the pharmaceutical point of view. Preferred excipients for their use in the present invention include sugars, starches, celluloses, gums and proteins. In a particular embodiment, the pharmaceutical composition of the invention will be formulated in a solid pharmaceutical dosage form (for example tablets, capsules, coated tablets, granules, suppositories, crystalline or amorphous sterile solids which can be reconstituted to provide liquid forms etc.), liquid pharmaceutical dosage form (for example solutions, suspensions, emulsions, elixirs, lotions, unguents etc.) or semisolid pharmaceutical dosage form (gels, ointments, creams and the like). The pharmaceutical compositions of the invention can be administered by any route including, without being limited to, oral, intravenous, intramuscular, intra-arterial, intramedullary, intrathecal, intraventricular, transdermal, subcutaneous, intraperitoneal, intranasal, enteral, topical, sublingual or rectal route. A review of the different forms of administration of active ingredients, of the excipients to be used and of the processes for manufacturing them can be found in Tratado de Farmacia Galénica, C. Faulí i Trillo, Luzán 5, S.A. de Ediciones, 1993 and in Remington's Pharmaceutical Sciences (A.R. Gennaro, Ed.), 20th edition, Williams & Wilkins PA, USA (2000) Examples of pharmaceutically acceptable vehicles are known in the state of the art and include phosphate-buffered saline solutions, water, emulsions, such as oil/water emulsions, different types of wetting agents, sterile solutions, etc. The compositions comprising said vehicles can be formulated by conventional processes known in the state of the art.

In the event that nucleic acids (the polynucleotides of the invention, the vectors or the gene constructs) are administered, the invention provides pharmaceutical compositions especially prepared for the administration of said nucleic acids. The pharmaceutical compositions can comprise said nucleic acids in naked form, i.e., in the absence of compounds protecting the nucleic acids from their degradation by the nucleases of the organism, which involves the advantage that the toxicity associated to the reagents used for the transfection is eliminated. Suitable routes of administration for the naked compounds include intravascular, intratumoral, intracranial, intraperitoneal, intrasplenic, intramuscular, subretinal, subcutaneous, mucosal, topical and oral route (Templeton, 2002, DNA Cell Biol., 21:857-867). Alternatively, the nucleic acids can be administered forming part of liposomes, conjugated to cholesterol or conjugated to compounds which can promote the translocation through cell membranes such as peptide Tat derived from the HIV-1 TAT protein, the third helix of the homeodomain of the D.melanogaster Antennapedia protein, the VP22 protein of the herpes simplex virus, arginine oligomers and peptides such as those described in WO07069090 (Lindgren, A. et al., 2000, Trends Pharmacol. Sci, 21:99-103, Schwarze, S.R. et al. , 2000, Trends Pharmacol. Sci., 21:45-48, Lundberg, M et al., 2003, Mol. Therapy 8:143-150 and Snyder, E.L. and Dowdy, S.F., 2004, Pharm. Res. 21:389-393). Alternatively, the polynucleotide can be administered forming part of a plasmid vector or of a viral vector, preferably vectors based on adenoviruses, in adeno-associated viruses or in retroviruses, such as viruses based on the murine leukemia virus (MLV) or in lentiviruses (HIV, FIV, EIAV).

In another embodiment, the compositions and polynucleotides of the invention are administered by means of the so-called "hydrodynamic administration", as has been described by Liu, F., et al., (Gene Ther, 1999, 6:1258-66). According to said method, the compounds are intravascularly introduced in the organism at a high rate and volume, which results in high transfection levels with a more diffused distribution. It has been demonstrated that the efficacy of the intracellular access depends directly on the volume of fluid administered and on the rate of the injection (Liu et al., 1999, Science, 305:1437-1441). In mice, the administration has been optimized in values of 1ml/10 g of body weight in a period of 3-5 seconds (Hodges et al., 2003, Exp.Opin.Biol.Ther, 3:91-918). The exact mechanism allowing *in vivo* cell transfection with polynucleotides after their hydrodynamic administration is not completely known. In the case of mice, it is believed that the administration through the tail vein takes place at a rate exceeding the heart rate, which causes the administered fluid to accumulate in the superior vena cava. This fluid subsequently accesses the vessels in the organs and, subsequently, through fenestrations in said vessels, it accesses the extravascular space. The polynucleotide thus comes into contact with the cells of the target organ before it is mixed with blood, thus reducing the possibilities of degradation by nucleases.

The compositions of the invention can be administered in doses of less than 10 mg per kilogram of body weight, preferably less than 5, 2, 1, 0.5, 0.1, 0.05, 0.01, 0.005, 0.001, 0.0005, 0,0001, 0.00005 or 0.00001 mg per kg of body weight and less than 200 nmol of RNA agent, i.e., about $4.4 \times 10^{16}$ copies per kg of body weight or less than 1500, 750, 300, 150, 75, 15, 7.5, 1.5, 0.75, 0.15 or 0.075 nmol per kg of body weight. The unit doses can be administered by injection, by inhalation or by topical administration. The bifunctional polynucleotides and compositions of the invention can be administered directly in the organ in which the target mRNA is expressed, in which case doses of between 0.00001 mg to 3 mg per organ, or preferably between 0.0001 and 0.001 mg per organ, about 0.03 and 3.0 mg per organ, about 0.1 and 3.0 mg per organ or between 0.3 and 3.0 mg per organ are administered.

The dose depends on the severity and response of the condition to be treated and can vary between several days and several months or until it is observed that the condition remits. The optimal dosage can be determined by carrying out periodic measurements of the concentrations of the agent in the organism of the patient. The optimal dose can be determined from the EC50 values obtained by means of prior in vitro or in vivo assays in animal models. The unit dose can be administered once a day or less than once a day, preferably less than once every 2, 4, 8 or 30 days. Alternatively, it is possible to administer an initial dose followed by one or several maintenance doses, generally of a smaller amount than the initial dose. The maintenance regimen can involve treating the patient with doses ranging between 0.01 μg and 1.4 mg/kg of body weight per day, for example 10, 1, 0.1, 0.01, 0.001, or 0.00001 mg per kg of body weight per day. The maintenance doses are preferably administered at most once every 5, 10 or 30 days. The treatment must be continued for a time period which will vary according to the type of disorder that the patient suffers from, its severity and the condition of the patient. After the treatment, the evolution of the patient must be monitored to determine if the dose must be increased in the event that the disease does not respond to the treatment or the dose is decreased if an improvement of the disease is observed or if undesirable side effects are observed.

The daily dose can be administered in a single dose or in two or more doses according to the particular circumstances. If a repeated administration or frequent administrations are desired, the implantation of an administration device such as a pump, a semi-permanent (intravenous, intraperitoneal, intracisternal or intracapsular) catheter or a reservoir is recommendable.

The conjugates of the invention, the polynucleotides encoding them, the gene constructs and vectors comprising said polynucleotides and the nanolipoparticles of the invention can be used in methods of therapeutic treatment given the capacity of said conjugates of carrying a compound of therapeutic interest to a target tissue. A person skilled in the art will understand that the diseases which can be treated with the compounds of the invention will depend (i) on the active component which is associated to Apo A and (ii) on the tissue to which said conjugates are carried. Table 2 describes, in a non-limiting manner, possible diseases which can be treated with said conjugates and the active ingredient which would have to be incorporated to the conjugate:

| Conjugate | Disease |
|---|---|
| *Apo-IFNα5* | chronic hepatitis C<br>chronic hepatitis B<br>adjuvant vaccines<br>hepatocarcinoma |
| *Apo-oncostatin* | chronic hepatitis C<br>chronic hepatitis B<br>hepatocarcinoma |
| *Apo-cardiotrophin* | Liver transplant<br>Kidney transplant<br>Hepatectomies |
| *Apo-IL6* | Liver transplant<br>Kidney transplant<br>Hepatectomies |
| *Apo-amphiregulin* | Liver transplant<br>Hepatectomies |
| *Apo-EDA*: | Vaccine adjuvant |
| *Apo-IL15* | Adjuvant in immunotherapy |
| *Apo-IL12* | Hepatocarcinoma |
| *Apo-CD134*: | Adjuvant in immunotherapy |
| *Apo-CD137*: | Adjuvant in immunotherapy |
| *Apo- PBGD*: | Acute intermittent porphyria |
| *Apo-p17(TGF-β1 inhibitor)* | Adjuvant in colon cancer<br>Pulmonary fibrosis<br>Bone metastasis |
| *Apo-p144(TGF-β1 inhibitor)* | Adjuvant in colon cancer<br>Breast prostheses<br>Systemic sclerosis<br>Morphea<br>Bums<br>Cardiac fibrosis<br>Renal fibrosis |
| *Apo- IL10 inhibitors* | Viral infections<br>Bacterial infections<br>Parasitic infections<br>Non-Hodgkin's lymphoma |
| *Apo-FoxP3 inhibitors* | Adjuvant in immunotherapy (regulatory T cells blocking) |
| *Apo-TNFα inhibitors* | Rheumatoid arthritis |
| *Apo-VEGF inhibitors* | Antiangiogenesis |
| *Apo-PD-1 inhibitors* | Adjuvant in immunotherapy |
| *Apo-CD152 inhibitors* | Adjuvant in immunotherapy |

The conjugates of the invention have the capacity to be targeted to the organs or tissues in which there is expression of surface molecules with sufficient affinity for ApoA and with the capacity to be internalized after the binding with said polypeptide. Said surface molecules include SR-B1 (scavenger receptor B type 1), SR-A1 (scavenger receptor A type 1), SR-A2 (scavenger receptor A type 1) and SR-C (scavenger receptor C). The therapeutically active compounds can thus be carried to said target organs or tissues. These organs include not only the liver, but also all the cells expressing on their surface sufficient amounts of the SR-BI receptor. Example 7 of the present invention thus illustrates the presence of the SR-BI receptor in different populations of the immune system and, in particular, in CD4+ T cells, in CD8+ T cells, in NK cells; in dendritic cells and in monocytes/macrophages. The invention thus also provides the use of the conjugates of the invention for the treatment of diseases associated to the immune system. Additionally, the expression of the SR-BI receptor in osteoclasts (Brodeur et al., 2008, J. Bone Miner Res. 23:326-37), in endothelial cells (Yeh et al., 2002, Atherosclerosis, 161:95-103), intestinal epithelium (Cai, S. F. et al., 2001, J. Lipid Res. 42: 902-909), in the bile duct epithelium (Miquel et al., Gut., 2003, 52:1017-1024), in adipose tissue (Acton et al., 1994, J.Biol.Chem., 269:21003-21009) and in the lung (Acton et al., 1994, J.Biol.Chem., 269:21003-21009) is known.

Therefore, the conjugates of the present invention are suitable for carrying compounds of therapeutic interest to the previously indicated compartments. Thus, considering the target organ, the conjugates of the invention can be used for the treatment of liver diseases such as intrahepatic cholestasis, fatty liver (alcoholic fatty liver, Reye's syndrome), hepatic vein thrombosis, hepatoventricular degeneration, hepatomegaly, hepatopulmonary syndrome, hepatorenal syndrome, portal hypertension, hepatic abscesses, cirrhosis (alcoholic, biliary, experimental cirrhosis), alcoholic liver diseases (fatty liver, hepatitis, cirrhosis), parasitic diseases (echinococcosis, fascioliasis, amebic abscesses), jaundice (hemolytic, hepatocellular and cholestatic), hepatitis (alcoholic hepatitis, chronic hepatitis, autoimmune hepatitis, hepatitis B, hepatitis C, hepatitis D, drug-induced hepatitis, toxic hepatitis, viral hepatitis (hepatitis A, B, C, D and E), Wilson's disease, granulomatous hepatosis, secondary biliary cirrhosis, primary biliary cirrhosis, hepatic encephalopathy, portal hypertension, hepatocellular adenoma, hemangioma, gallstones, hepatic neoplasms (angiomyolipoma, calcified liver metastases, cystic liver metastases, fibrolamellar hepatocarcinoma, focal nodular hyperplasia, hepatic adenoma, hepatobiliary cystadenoma, hepatoblastoma, hepatocellular carcinoma, hepatoma, liver cancer, hepatic hemangioendothelioma, regenerative nodular hyperplasia, benign liver tumors, hepatic cysts (simple cysts, polycystic cysts, hepatobiliary cystadenoma, mesenchymal liver tumors [mesenchymal hamartoma, infantile hemangioendothelioma, hemangioma, peliosis hepatis, lipomas, inflammatory pseudotumor], epithelial bile duct tumors, bile duct hamartoma, bile duct adenoma, malignant liver tumors [hepatocellular, hepatoblastoma, hepatocellular carcinoma, cholangiocellular cancer, cholangiocarcinoma, cystadenocarcinoma, capillary tumors, angiosarcoma, Kaposi's sarcoma, hemangioendothelioma, embryonal sarcoma, fibrosarcoma, leiomyosarcoma, rhabdomyosarcoma, carcinosarcoma, teratoma, squamous carcinoma, primary lymphoma]), erythrohepatic porphyria, hepatic porphyria (acute intermittent porphyria, late cutaneous porphyria), Zellweger syndrome.

The conjugates of the invention can be used for the treatment of immune system diseases such as:

- autoimmune diseases: Addison's disease, autoimmune hemolytic anemia, anti-glomerular basement membrane antibody disease, antiphospholipid syndrome, rheumatoid arthritis, autoimmune nervous system diseases, dermatitis herpetiformis, type 1 diabetes mellitus, familial Mediterranean fever, IGA glomerulonephritis, membranous glomerulonephritis, Goodpasture's syndrome, Graves' disease, autoimmune hepatitis, Lambert-Eaton's myasthenic syndrome, systemic lupus erythematosus, sympathetic ophthalmia, pemphigus, autoimmune polyendocrinopathies, idiopathic thrombocytopenic purpura, Reiter's disease and autoimmune thyroiditis),
- diseases due to blood group incompatibility: erythroblastosis fetalis, Rh isoimmunization,
- membranoproliferative glomerulonephritis,
- graft-versus-host disease,
- hypersensitivity: hypersensitivity to drugs, environmental diseases, retarded hypersensitivity (cell migration inhibition, acute disseminated encephalomyelitis), immediate hypersensitivity (anaphylaxis, allergic conjunctivitis, atopic dermatitis), immune complex diseases (vasculitis due to hypersensitivity, Arthus reaction, serum sickness), hypersensitivity to latex, Wissler's syndrome.
- Immunological deficiency syndromes such as dysgammaglobulinemia, HIV-1 infections, HTLV-1 or HTLV-2 infections, enzootic bovine leukosis, lymphopenia, phage dysfunctions such as Chediak-Higashi syndrome, chronic granulomatous disease, Job syndrome, agammaglobulinemia, ataxia telangiectasia, common variable immunodeficiency, DiGeorge syndrome, leukocyte adhesion deficiency syndrome, Wiskott-Aldrich syndrome,
- thrombocytopenic purpura,
- immunoproliferative disorders: hyperglobulinemia (Schnitzler's syndrome), lymphoproliferative disorders (granuloma, heavy chain disease, hairy cell leukemia, lymphocytic leukemia, myeloid leukemia, lymphangiomyoma, lymphoma, sarcoidosis, agammaglobulinemia, giant lymph node hyperplasia, immunoblastic lymphadenopathy, infectious mononucleosis, lymphomatoid granulomatosis, Marek's disease, Sézary syndrome, tumor lysis syndrome, Waldenström's macroglobulinemia, immunoproliferative small intestine disease, plasmacytic leukemia, paraproteinemias and thrombocytopenic purpura), paraproteinemias.

The conjugates of the invention can be used for the treatment of capillary endothelium diseases such as arteriosclerosis, obliterative arteriopathy, Raynaud's disease due to connectivitis, primitive hypertension and secondary pulmonary hypertension, diabetic microangiopathy, Buerger's disease, systemic sclerosis, vasculitis and all the diseases characterized by endothelial damage with the subsequent ischemia.

The conjugates of the invention can be used for the treatment of bone diseases such as dysplasias characterized by an abnormal bone growth. Representative examples of such conditions are achondroplasia, cleidocranial dysostosis, enchondromatosis, fibrous dysplasia, Gaucher's disease, hypophosphatemic rickets, Marfan syndrome, hereditary multiple exostoses, neurofibromatosis, osteogenesis imperfecta, osteopetrosis, osteopoikilosis, sclerotic lesions, fractures, periodontal disease, pseudoarthrosis, pyogenic osteomyelitis, conditions resulting in osteopenia such as anemic conditions, osteopenia caused by steroids and heparin, bone marrow disorders, scurvy, malnutrition, calcium deficiency, idiopathic osteoporosis, congenital osteopenia, alcoholism, Cushing's disease, acromegaly, hypogonadism, transient regional osteoporosis and osteomalacia.

The conjugates of the invention can be used for the treatment of intestinal epithelium diseases such as malabsorption syndromes, Crohn's disease, intestinal diverticular disease, paralytic ileus and intestinal obstruction.

The conjugates of the invention can be used for the treatment of respiratory diseases such as nasal vestibulitis, non-allergic rhinitis (for example, acute rhinitis, chronic rhinitis, atrophic rhinitis, vasomotor rhinitis), nasal polyps, sinusitis, juvenile angiofibromas, nose cancer and juvenile papillomas, vocal cord polyps, nodules, contact ulcers, vocal cord paralysis, laryngoceles, pharyngitis, tonsillitis, tonsillar cellulitis, parapharyngeal abscesses, laryngitis, laryngocele, throat cancer (for example, nasopharyngeal cancer, tonsil cancer, larynx cancer), lung cancer (squamous cell carcinoma, microcytic carcinoma, macrocytic carcinoma, adenocarcinoma), allergic disorders (eosinophilic pneumonia, allergic alveolitis, allergic interstitial pneumonia, allergic bronchopulmonary aspergillosis, asthma, Wegener's granulomatosis, Goodpasture's syndrome, pneumonia (for example, bacterial pneumonia (for example, that caused by *Streptococcus pneumoniae,* by *Staphylococcus aureus*, by Gram-negative bacteria such as *Klebsiella* and *Pseudomonas spp,* that caused by *Mycoplasma pneumoniae,* by *Haemophilus influenzae,* by *Legionella pneumophil* and by *Chlamydia psittaci* and viral pneumonias (for example, influenza or chicken pox), bronchiolitis, polio, laryngotracheobronchitis (also called croup syndrome), respiratory infection due to syncytial viruses, mumps, erythema infectiosum, roseola infantum, rubella, fungal pneumonia, (for example histoplasmosis, coccidioidomycosis, blastomycosis and fungal infections in immunosuppressed patients such as cryptococcosis caused by *Cryptococcus neoformans*; aspergillosis caused by *Aspergillus spp*.; candidiasis, caused by *Candida*; and mucormycosis), infection due to *Pneumocystis carinii,* atypical pneumonias (for example, those caused by *Mycoplasma* and *Chlamydia* spp.), opportunistic pneumonia, nosocomial pneumonia, chemical pneumonitis and aspiration pneumonia, pleural disorders (for example pleurisy, pleural effusion and pneumothorax (for example simple spontaneous pneumothorax, complex spontaneous pneumothorax, tension pneumotorax), obstructive respiratory tract disease (for example asthma, chronic obstructive pulmonary disease, emphysema, chronic or acute bronchitis), occupational pulmonary diseases (for example silicosis, black lung disease, asbestosis, berylliosis, occupational asthma, byssinosis and benign pneumoconiosis), infiltrative pulmonary disease such as pulmonary fibrosis, fibrosing alveolitis, idiopathic pulmonary fibrosis, desquamative interstitial pneumonia, lymphoid interstitial pneumonia, histiocytosis X (for example Letterer-Siwe disease, Hand-Schuller-Christian disease, eosinophilic granuloma), idiopathic pulmonary hemosiderosis, sarcoidosis and pulmonary alveolar proteinosis, acute respiratory distress syndrome, edema, pulmonary embolism, bronchitis (for example, viral, bacterial bronchitis), bronchiectasis, atelectasis, lung abscesses and cystic fibrosis.

In a preferred embodiment, the invention contemplates that the therapeutically active component is an interferon. In that case, the conjugates or the polynucleotides encoding them will be useful for the treatment of liver diseases responding to interferon, such as chronic hepatitis C, chronic hepatitis B, hepatocarcinoma, cirrhosis, fibrosis.

In addition, given the presence of SR-BI receptors in immune system cells, the conjugates of the invention can be used to target therapeutically active compounds to said cells. Thus, in a preferred embodiment, the conjugates of the invention containing interferon as the therapeutically active compound can be used as an adjuvant for enhancing the immune response of a vaccine. The vaccine can be a vaccine targeted against an organism capable of triggering an infectious disease, a vaccine targeted against a tumor or a vaccine targeted against an allergen. The vaccines can contain a component of an infectious agent, a tumor or an allergen (peptide, polypeptide, glycopeptide, multiepitope peptide, fragment, etc.) or can be a gene vaccine formed by a nucleic acid encoding a polypeptide of said organism, tumor or allergen.

In another embodiment, the conjugates of the invention comprise a TGF-β1 peptide inhibitor. These conjugates can be used in the treatment of diseases or pathological disorders associated to an excess or deregulated expression of TGF-β1, such as (i) fibrosis associated with the loss of function of an organ or a tissue, for example, pulmonary fibrosis, hepatic fibrosis (cirrhosis), cardiac fibrosis, renal fibrosis, corneal fibrosis, etc., as well as (ii) surgical and aesthetic complications, for example, fibrosis associated with cutaneous and peritoneal surgery, fibrosis associated with burns, osteoarticular fibrosis, keloids, etc.

The authors of the present invention have shown that the administration of the conjugates of the invention comprising a TGF-β1 peptide inhibitor together with IL-12 results in a stimulation of the induction of IFN-gamma mediated

**EP 2 305 309 A2**

by IL-12. Given that IFN-γ is a known antitumor agent, the findings of the inventors opens up the way for a new antitumor treatment based on the combination of an immunostimulating cytokine and to a conjugate according to the invention comprising a TGF-β1 inhibitor peptide.

Therefore, in another aspect, the invention relates to a composition comprising

(a) a first component selected from the group of a conjugate, a polynucleotide, a gene construct, a vector, a host cell, a nanolipoparticle and a paharmaceutical composition according to the invention wherein component (ii) is a TGF-β1 inhibitor peptide and

(b) a second component selected from the group of a an immunostimulatory cytokine, a polynucleotide encoding said cytokine, a vector comprising said polynucleotide, a TGF-β1 inhibitor peptide, a cytotoxic agent and combinations thereof.

Immunostimulating cytokines which can be administered together with the Apo A conjugates comprising the TGF-β1 inhibitor peptides include, but are not limited to, IL-12, IL-2, IL-15, IL-18, IL-24, GM-CSF, TNF-α, CD40 ligand, IFN-α, IFN-β, IFN-γ. In a preferred embodiment, the stimulating cytokine is IL-12.

TGF-β1 inhibitor peptides that may form component (b) of the composition of the invention are essentially the same as those that form part of the conjugate of the invention as described previously. Thus, the TGF-β1 inhibitor peptides may be, without limitation, peptide p144 (SEQ ID NO:4) or peptide p17 (SEQ ID NO:5). The peptides forming part of the conjugate and forming the second component of the invention may be the same or different.

A "cytotoxic agent", as used herein, is a compound capable of selectively or non-selectively killing or inhibiting the growth of a cell. Examples include paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, epirubicin, and cyclophosphamide and analogs or homologs thereof, antimetabolites (e.g., methotrexate, 6- mercaptopurine, 6-thioguanine, cytarabine, 5-fluorouracil decarbazine), alkylating agents (e.g., mechlorethamine, thioepa chlorambucil, melphalan, carmustine (BCNU) and lomustine (CCNU), busulfan, dibromomannitol, streptozotocin, mitomycin C, and cisdichlorodiamine platinum (II) (DDP) cisplatin), anthracyclines (e.g., daunorubicin and doxorubicin), antibiotics (e.g., dactinomycin), bleomycin.

In another aspect, the compositions of the invention can be used for the treatment of different types of tumors including, but not limited to, hematological cancers (leukemias or lymphomas, for example), neurological tumors (astrocytomas or glioblastomas, for example), melanoma, breast cancer, lung cancer, head and neck cancer, gastrointestinal tumors (stomach, pancreas or colon cancer, for example), liver cancer (for example, hepatocellular carcinoma), renal cell cancer, genitourinary tumors (ovarian cancer, vaginal cancer, cervical cancer, bladder cancer, testicle cancer, prostate cancer, for example) bone tumors and vascular tumors.

Thus, in another aspect, the invention relates to a composition of the invention for the treatment of cancer. In another aspect, the invention relates to a method for the treatment of cancer which comprises the administration to a subject in need thereof of a composition according to the invention. In another aspect, the invention relates to the use of a composition of the invention for the preparation of a medicament for the treatment of cancer.

The therapeutically effective amounts of the components of the composition of the invention as described herein to be used will depend, for example, upon the therapeutic objectives, the route of administration, and the condition of the patient. Accordingly, it is preferred for the therapist to titer the dosage and modify the route of administration as required to obtain the optimal therapeutic effect. A typical daily dosage might range from about 0.01mg/kg to up to 250mg/kg or more, daily, every 2 days, every 3 days, every 4 days, every 5 days, every 6 days or weekly.

Administration of the composition may carried out by different means. For example components (a) and (b) of the composition may be administered sequentially, separately and/or simultaneously. In one embodiment, components (a) and (b) of the composition are administered simultaneously (optionally repeatedly). In one embodiment the separate formulations are administered sequentially (optionally repeatedly). In one embodiment the separate formulations separately (optionally repeatedly). The skilled person will understand that where the separate formulations of components (a) and (b) are administered sequentially or serially, that this could be administration of component (a) followed by component (b) or component (b) followed by component (a). In one embodiment the separate formulations of components (a) and (b) may be administered in alternative dosing patterns. Where the administration of the separate formulations of components (a) and (b) of the composition of the invention is sequential or separate, the delay in administering the second formulation should not be such as to lose the beneficial effect of the combination therapy.

The invention is illustrated below based on the following examples which are provided by way of a non-limiting illustration of the scope of the invention.

**EXAMPLES**

**EXAMPLE 1**

MATERIALS AND METHODS

**1. Construction of the expression vectors:**

1.1 RNA extraction:

Total RNA from mice liver or from brain of treated mice was isolated from individual samples using TRI reagent (Sigma, Madrid, Spain). The concentration and purity of the samples were determined by the absorbance at 260 and 280nm with background correction at 320nm in a spectrophotometer (*Biophotometer,* Eppendorf).

1.2 RT-PCR synthesis of total cDNA:

The total RNA (3μg) was treated with DNase I and retrotranscribed to cDNA with M-MLV RT in the presence of RNase OUT (all the reagents were from Invitrogen, Carlsbed, CA). 25 μl of liver total cDNA were obtained. The reaction was incubated for 1 hour at 37˚C, denatured for 1 minute at 95˚C and taken to 4˚C. The samples were used immediately for PCR or stored at -20˚C.

1.3 Obtaining and cloning murine Apolipoprotein A1(mApoA1) cDNA:

The sense primer 5'-ATGAAAGCTGTGGTGCTGGC-3' (FwATGmApoA1) (SEQ ID NO: 20) and the antisense primer 5'-TCACTGGGCAGTCAGAGTCT-3' (RvTGAmApoA1) (SEQ ID NO: 21) were designed. The mApoA1 cDNA (795 total nucleotides, 72 nucleotides encoding the signal peptide and 723 nucleotides encoding the native protein) was amplified by means of PCR on the liver total cDNA, using BioTaq DNA polymerase (Bioline, London, United Kingdom): 5 minutes at 94˚C, 30 cycles of 40 seconds at 94˚C, 40 seconds at 55˚C and 40 seconds at 72˚C, followed by 7 minutes at 72˚C in a *2720 Thermal cycler* (Applied Biosystems, Foster City, USA). The PCR product was migrated in an Agarose D-1 low EEO 1% agarose gel (Pronadisa, Madrid, Spain), and the gel fragment was purified by means of a QIAquick Gel Extraction Kit (Qiagen, Valencia, CA). The purified cDNA of mApoA1 was cloned, according to the instructions provided by the manufacturer, into the expression vector pcDNA™3.1/V5-His TOPO® TA (Invitrogen, Carlsbed, CA), which will be called pCMV-mApoA1. Finally, the sequence obtained was confirmed by means of sequencing.

1.4 Obtaining and cloning of murine Interferon alpha 1 (mIFNα1) cDNA:

The sense primer 5'-ATGGCTAGGCTCTGTGCTTT-3' (FwATGmIFNα1) (SEQ ID NO: 22) and the antisense primer 5'-TCATTTCTCTTCTCTCAGTC-3' (RvTGAmIFNα1) (SEQ ID NO:23) were designed. The mIFNα1 cDNA (570 total nucleotides, 69 nucleotides encoding the signal peptide and 501 nucleotides encoding the native protein) was amplified by means of PCR on the liver total cDNA, using BioTaq DNA polymerase (Bioline, London, United Kingdom). The amplification conditions were: 5 minutes at 94˚C, 30 cycles of 40 seconds at 94˚C, 40 seconds at 55˚C and 40 seconds at 72˚C, followed by 7 minutes at 72˚C in a 2720 Thermal cycler (Applied Biosystems Foster City, USA). The PCR product was migrated in an Agarose D-1 low EEO 1% agarose gel (Pronadisa, Madrid, Spain), and the gel fragment was purified by means of a QIAquick Gel Extraction Kit (Qiagen, Valencia, CA). The purified cDNA of mIFNα1 was cloned, according to the provided instructions, into the expression vector *pcDNA™3.1/V5-His TOPO® TA* (Invitrogen, Carlsbed, CA), which will be called pCMV-mIFNα1. Finally, the sequence was confirmed by means of sequencing.

1.5 Gene fusion design:

*1.5.1 C-terminalfusion of the mIFNα1 gene to the mApoA1 gene: Apo-IFN*

The antisense primer 5'-GGCGCGCCCTGGGCAGTCAGAGTCTCGC-3' (RvAsclmApoA1) (SEQ ID NO:24) was designed, which introduces the 9-nucleotide sequence (GGCGCGCCC) which forms a restriction site for the Ascl enzyme in 3' of the ApoA1 gene and eliminates the stop codon. This added restriction sequence will be translated into a short binding peptide GAP, which will provide certain mobility to the constituent proteins. The sense primer 5'GGCGCGCCCTGTGACCTGCC TCAGACTCA-3' (FwAsclmIFNα1) (SEQ ID NO:25) was designed, which introduces the Ascl restriction sequence in 5' of the sequence encoding the mature mIFNα1 protein (i.e., elimination of the signal peptide sequence).

Amplification was carried out by PCR, using pCMV-mApoA1 as a template, and the primers FwATGmApoA1 and RvAscImApoA1, with the BioTaq DNA polymerase enzyme (Bioline, London, United Kingdom), 5 minutes at 94˚C, 30 cycles of 40 seconds at 94˚C, 40 seconds at 57˚C and 40 seconds at 72˚C, followed by 7 minutes at 72˚C in a 2720 Thermal cycler (Applied Biosystems Foster City, USA). The PCR product (804 nucleotides) was migrated in an Agarose D-1 low EEO 1% agarose gel (Pronadisa, Madrid, Spain), and the gel fragment was purified by means of a QIAquick Gel Extraction Kit (Qiagen, Valencia, CA). The purified DNA of mApoA1-AscI was cloned, according to the provided instructions, into the expression vector pcDNA™3.1/V5-His TOPO® TA (Invitrogen, Carlsbed, CA), which will be called pCMV-mApoA1-AscI. Finally, the sequence was confirmed by means of sequencing.

In parallel, amplification was carried out by PCR using pCMV-mIFN$\alpha$1 as a template and the primers FwAscImIFN$\alpha$1 and RvTGAmIFN$\alpha$1. The BioTaq DNA polymerase enzyme (Bioline, London, United Kingdom) and the following amplification conditions were used: 5 minutes at 94˚C, 30 cycles of 40 seconds at 94˚C, 40 seconds at 57˚C and 40 seconds at 72˚C, followed by 7 minutes at 72˚C in a 2720 Thermal cycler (Applied Biosystems Foster City, USA). The PCR product (510 nucleotides) was migrated in an Agarose D-1 low EEO 1% agarose gel (Pronadisa, Madrid, Spain), and the gel fragment was purified by means of a *QIAquick Gel Extraction Kit* (Qiagen, Valencia, CA). The purified DNA of AscI-mIFN$\alpha$1 was cloned, according to the provided instructions, into the expression vector pcDNA™3.1/V5-His TOPO® TA (Invitrogen, Carlsbed, CA), which will be called *pCMV-AscI-mIFN$\alpha$1.* Finally, the sequence was confirmed by means of sequencing.

To carry out the gene fusion, the plasmids *pCMV-mApoA1-AscI and pCMV-AscI-mIFN$\alpha$1* were digested independently for 1.5 hours at 37˚C with the AscI/PmeI enzymes, 1xBSA and Buffer 4 (New England Biolabs), using the restriction site PmeI present in the pcDNA 3.1 V5-His TOPO® TA skeleton. Both digestions were migrated in 1% agarose gel and the corresponding bands were purified to the open vector pCMV-mApoA1-AscI and to the pCMV-AscI-mIFN$\alpha$1 insert. The product was ligated in a 1:3 (vector:insert) ratio using T4 DNA ligase High Concentration and a 2X Rapid Ligation Buffer (Promega Madison, W1, USA) as a buffer solution, incubating the mixture for 10 minutes at room temperature. Top10 bacteria (Invitrogen, Carlsbed, CA) were subsequently transformed. The transformed bacteria were selected by their growth in Petri dishes with LB medium with ampicillin, since the vector contains a gene resistant to this antibiotic. The plasmid DNA of the positive bacteria was extracted by means of the MiniPrep technique (Qiagen, Germany) to subsequently digest 2 $\mu$g of said plasmid with the AscI/PmeI enzymes (New England Biolabs) and separate by electrophoresis the result of said digestion in 1% agarose gel to verify the presence of the insert. The resulting 6825 nt plasmid will hereinafter be called pCMV-Apo-IFN (pCMV-AF).

*1.5.2 N-terminal fusion of the mIFN$\alpha$1 gene to the mApoA1 gene: IFN-Apo*

The antisense primer 5'-GGGCGCGCCTTTCTCTTCTCTCAGTCTTC-3' (RvAscImIFN$\alpha$1) (SEQ ID NO:26) was designed, which introduces the 9-nucleotide sequence (GGCGCGCCC) which forms a restriction site for the AscI enzyme in 3' of the mIFN$\alpha$1 gene and eliminates the stop codon. The sense primer 5'-CCAGGCGCGCCGGATGAACCCCAGTCCCAATG-3' (FwAscImApoA1) (SEQ ID NO:27) was designed, which introduces the AscI restriction sequence in 5' of the sequence encoding the mature mApoA1 protein (i.e., elimination of the signal peptide sequence). This primer includes 3 nucleotides in 5' to allow the cleavage with AscI.

Amplification was carried out by PCR using pCMV-mApoA1 as a template, and the primers FwAscImApoA1 and RvTGAmApoA1, with the BioTaq DNA polymerase enzyme (Bioline, London, United Kingdom), using the following amplification conditions: 5 minutes at 94˚C, 30 cycles of 40 seconds at 94˚C, 40 seconds at 57˚C and 40 seconds at 72˚C, followed by 7 minutes at 72˚C in a 2720 Thermal cycler (Applied Biosystems Foster City, USA). The PCR product (732 nucleotides) was migrated in an Agarose D-1 low EEO 1% agarose gel (Pronadisa, Madrid, Spain), and the gel fragment was purified by means of a QIAquick Gel Extraction Kit (Qiagen, Valencia, CA). The purified DNA of AscI-mApoA1 was cloned, according to the provided instructions, into the expression vector *pcDNA™3.1/V5-His TOPO® TA* (Invitrogen, Carlsbed, CA), which will be called pCMV-AscI-mApoA1. Finally, the sequence was confirmed by means of sequencing.

In parallel, amplification was carried out by using PCR pCMV-mIFN$\alpha$1 as a template and the primers FwATGmIFN$\alpha$1 and RvAscImIFN$\alpha$1 with the BioTaq DNA polymerase enzyme (Bioline, London, United Kingdom) and the following conditions: 5 minutes at 94˚C, 30 cycles of 40 seconds at 94˚C, 40 seconds at 57˚C and 40 seconds at 72˚C, followed by 7 minutes at 72˚C in a 2720 Thermal cycler (Applied Biosystems Foster City, USA). The PCR product (576 nucleotides) was migrated in an Agarose D-1 low EEO 1% agarose gel (Pronadisa, Madrid, Spain), and the gel fragment was purified by means of a *QIAquick Gel Extraction Kit* (Qiagen, Valencia, CA). The purified DNA of mIFN$\alpha$1-AscI was cloned, according to the provided instructions, in the expression vector pcDNA™3.1/V5-His TOPO® TA (Invitrogen, Carlsbed, CA), which will be called pCMV-mIFN$\alpha$1-AscI. Finally, the sequence was confirmed by means of sequencing.

To carry out the gene fusion, plasmids pCMV-AscI-mApoA1 and pCMV-mIFN$\alpha$1-AscI were digested independently for 1.5 hours at 37˚C with the AscI/PmeI enzymes, 1xBSA and Buffer 4 (New England Biolabs), using the restriction site PmeI present in the pcDNA 3.1 V5-His TOPO® TA skeleton. Both digestions were migrated in a 1% agarose gel and the corresponding bands were purified to the open vector pCMV-mIFNα1-AscI and to the pCMV-AscI-mApoA1 insert. The product was ligated in a 1:3 (vector:insert) ratio using T4 DNA ligase High Concentration and 2X Rapid Ligation Buffer (Promega Madison, W1, USA) as a buffer solution, incubating the mixture for 10 minutes at room temperature. Top10 bacteria (Invitrogen, Carlsbed, CA) were subsequently transformed. The transformed bacteria were selected by their growth in Petri dishes with LB medium with ampicillin, since the vector contains a gene resistant to this antibiotic. The plasmid DNA of positive bacteria was extracted by means of the MiniPrep technique (Qiagen, Germany) to subsequently digest 2 μg of said plasmid with the AscI/PmeI enzymes (New England Biolabs) and separate by electrophoresis the result of said digestion in a 1% agarose gel to verify the presence of the insert. The resulting 6822-nucleotide plasmid will hereinafter be called pCMV-IFN-Apo (pCMV-IA).

*1.5.3 C-terminalfusion of peptide p17 to the mApoA1 gene*:

The primer 5'-TCACGCACGCTCATACCAAGAACTCCTAGGAATAAACCAAATACGCTTGGG CGCGCCCTGGGC-3' (RvmApoA1p17) (SEQ ID NO:28) was designed. It was amplified by PCR, with the Easy-A High Fidelity PCR cloning enzyme (Stratagene, La Jolla, CA) using pCMV-mApoA1-AscI as a template and the primers FwATGmApoA1 and RvmApoA1p17. The following conditions were used: 1 minute at 95˚C, 30 cycles of 40 seconds at 95˚C, 40 seconds at 60˚C and 1 minute at 72˚C, followed by 7 minutes at 72˚C in a 2720 Thermal cycler (Applied Biosystems Foster City, USA). The PCR product was migrated in an Agarose D-1 low EEO 1% agarose gel (Pronadisa, Madrid, Spain), and the gel fragment was purified by means of a QIAquick Gel Extraction Kit (Qiagen, Valencia, CA). The purified DNA of mApoA1-AscI-p17 was cloned, according to the provided instructions, in the expression vector pcDNA™3.1/V5-His TOPO® TA (Invitrogen, Carlsbed, CA), which will be called pCMV-mApoA1-AscI-p 17. Finally, the sequence was confirmed by means of sequencing.

*1.5.4 C-terminalfusion of peptide p144 to the mApoA1 gene*:

The primer 5'-TCAATTCTGCATCATGGCCCAGATTATCGAGGCGTCCAGCGAGGTGGGCGC GCCCTGGGC-3' (RvmApoA1p144) (SEQ ID NO:29) was designed. It was amplified by PCR, with the Easy-A High Fidelity PCR cloning enzyme (Stratagene, La Jolla, CA) using pCMV-mApoA1-AscI as a template and the primers FwATGmApoA1 and RvmApoA1p144. The following conditions were used: 1 minute at 95˚C, 30 cycles of 40 seconds at 95˚C, 40 seconds at 65˚C and 1 minute at 72˚C, followed by 7 minutes at 72˚C in a 2720 Thermal cycler (Applied Biosystems Foster City, USA). The PCR product was migrated in an Agarose D-1 low EEO 1% agarose gel (Pronadisa, Madrid, Spain), and the gel fragment was purified by means of QIAquick Gel Extraction Kit (Qiagen, Valencia, CA). The purified DNA of mApoA1-AscI-p144 was cloned, according to the provided instructions, in the expression vector *pcDNA™3.1/V5-His TOPO® TA* (Invitrogen, Carlsbed, CA), which will be called pCMV-mApoA1-AscI-p 144. Finally, the sequence was confirmed by means of sequencing.

*1.5.5 Cloning the gene sequence of the mApoA1 signal peptide*:

To construct plasmids which will serve as a control in the fusion mApoA1 and peptide fusion experiments, the gene fusion of the mApoA1 signal peptide sequence (SPmApoA1) with peptides p17 and p144 is carried out (without adding the sequence for AscI), and thus ensuring that the secretion of the peptide is the same as that of mApoA1-peptides. The primer 5'-TTGCTGCCATACGTGCCAAG-3' (RvSPmApoA1) (SEQ ID NO:30) was designed, which together with the primer FwATGmApoA1 is used to amplify SPmApoA1, using pCMV-mApoA1 as a template. The PCR product (72 nucleotides) was migrated in an Agarose D-1 low EEO 1% agarose gel (Pronadisa, Madrid, Spain), and the gel fragment was purified by means of QIAquick Gel Extraction Kit (Qiagen, Valencia, CA). The purified DNA of SPmApoA1 was cloned, according to the provided instructions, in the expression vector pcDNA™3.1/V5-His TOPO® TA (Invitrogen, Carlsbed, CA), which will be called pCMV-SPmApoAl. Finally, the sequence was confirmed by means of sequencing.

*1.5.6 C-terminalfusion of peptide p17 to the mApoA1 signal peptide gene sequence*:

The primer 5'-TCACGCACGCTCATACCAAGAACTCCTAGGAATAAACCAAATACGCTTTTG CTGCCA-GAAATGCCG-3' (RvSPmApoA1p) (SEQ ID NO:31) was designed, which together with the primer FwATGmApoA1 was used to amplify SPmApoA1-p17, starting from the pCMV-mApoA1 template. The PCR product (120 nucleotides) was migrated in an Agarose D-1 low EEO 1% agarose gel (Pronadisa, Madrid, Spain), and the gel fragment was purified by means of QIAquick Gel Extraction Kit (Qiagen, Valencia, CA). The purified DNA of SPmApoA1-p17 was cloned, according to the provided instructions, in the expression vector pcDNA™3.1/V5-His TOPO® TA (Invitrogen, Carlsbed, CA), which will be called pCMV SPmApoA1-p17. Finally, the sequence was confirmed by means of sequencing.

*1.5.7 C-terminalfusion of peptide p144 to the mApoA1 signal peptide gene sequence*

The primer 5'-TCATTCTGCATCATGGCCCAGATTATCGAGGCGTCCAGCGAGGTTTGCTGCC AGAAATGCCG-3' (RvSPmApoA1p144) (SEQ ID NO:32) was designed, which together with the primer FwATGmApoA1 was used to amplify SPmApoA1-p144, starting from the pCMV-mApoA1 template. The PCR product (117 nucleotides) was migrated in an Agarose D-1 low EEO 1% agarose gel (Pronadisa, Madrid, Spain), and the gel fragment was purified by means of QIAquick Gel Extraction Kit (Qiagen, Valencia, CA). The purified DNA of SPmApoA1-p144 was cloned, according to the provided instructions, in the expression vector pcDNA™3.1/V5-His TOPO® TA (Invitrogen, Carlsbed, CA), which will be called pCMV-SPmApoA1-p144. Finally, the sequence was confirmed by means of sequencing.

*1.5.8 Introduction of MMP9 sequence in pCMV-mApoA1-AscI-p144*:

For the purpose of providing the fusion protein generated from this gene with the possibility of release by the cleavage of peptide p144, the proteases capable of cleaving the amino acid sequence of the fusion protein, releasing the complete p144 were studied by means of the MEROPS database (http://merops.sanger.ac.uk/). The result of the search gave as a candidate metalloprotease-9 (MMP9), which carried out the cleavage leaving an amino acid S in the p144 sequence. The use of this protease further conferred to the construct the capacity of release of this TGF-β inhibitor in the sites where its inhibition is necessary (localized, and not systemic, inhibition), since its presence in carcinomas has been described. The DNA sequence CTTTTCCCGACGTCT (SEQ ID NO:51) (amino acids: LFPTS, SEQ ID NO: 19) will be translated into said cleavage site: LFPT-S TSLDASIIWAMMQN (SEQ ID NO:4).

The primers 5'-CCAGGCGCGCCGCTTTTCCCGACGTCTACCTCGCTGGACGCCTC-3' (FwMMp9AscIp144) (SEQ ID NO:33) and 5'-TCAATTCTGCATCATGGCCCA-3' (RvMMp9AscIp144) (SEQ ID NO:34) were designed. Amplification was carried out by means of the Easy-A High Fidelity PCR cloning enzyme (Stratagene, La Jolla, CA), using pCMV-SPmApoA1-p144 as a template. The PCR was performed with the following conditions: 2 minutes at 94˚C, 30 cycles of 40 seconds at 94˚C, 45 seconds at 54˚C and 40 seconds at 72˚C, followed by 7 minutes at 72˚C in a 2720 Thermal cycler (Applied Biosystems Foster City, USA). The PCR product was migrated in an Agarose D-1 low EEO 1% agarose gel (Pronadisa, Madrid, Spain), and the gel fragment was purified (70 nucleotides), PerfectPrep DNA Cleanup (Eppendorf, Germany). The purified DNA of DNA AscI-MMP9-p144 was cloned, according to the instructions provided by the manufacturer, in the expression vector pcDNA™3.1/V5-His TOPO® TA (Invitrogen, Carlsbed, CA), which will be called pCMV-AscI-MMP9-p144. Finally, the sequence was confirmed by means of sequencing.

To carry out the gene fusion, plasmids pCMV-mApoA1-AscI-p144 and pCMV-AscI-MMp9-p144 were digested independently with the AscI/PmeI restriction enzymes, the latter being present in the pcDNA 3.1 V5-His TOPO® TA skeleton. The digestion was performed for 1.5 hours at 37˚C with the AscI/PmeI enzymes, 1xBSA and Buffer 4 (New England Biolabs, Beverly, USA). Both digestions were migrated in a 1% agarose gel and the corresponding bands were purified to the open vector pCMV-mApoA1-AscI-p144 and to the pCMV-AscI-MMp9-p1441 insert. The product was ligated in a 1:3 (vector:insert) ration using T4 DNA ligase High Concentration and 2X Rapid Ligation Buffer (Promega Madison, W1, U.S A) as a buffer solution, incubating the mixture for 10 minutes at room temperature. Top10 bacteria (Invitrogen, Carlsbed, CA) were subsequently transformed. The transformed bacteria were selected by their growth in Petri dishes with LB medium with ampicillin, since the vector contains a gene resistant to this antibiotic. The plasmid DNA of the positive bacteria was extracted by means of the MiniPrep technique (Qiagen, Germany) to subsequently digest 2 μg of said plasmid with the AccI/Pmel enzymes (New England Biolabs) and separate by electrophoresis the result of said digestion in a 1% agarose gel to verify the presence of the insert. The resulting 6324-nucleotide plasmid will hereinafter be called pCMV-mApoA1-AscI-MMP9-p144.

*1.5.9 Introduction of linker sequence in pCMV-mApoA1-p144*:

For the purpose of providing the fusion protein generated from this gene with the possibility of movements between the protein and peptide p144, the DNA sequence (GCACCAGCAGAAACAAAAGCAGAACCAATGAC, SEQ ID NO:53) encoding a flexible extended linker the sequence of which translated to amino acids is APAETKAEPMT (SEQ ID NO:13) was introduced, which adopts a CCCCCCCCCCC (coil) structure, and exists as a binding peptide in the native pyruvate ferredoxin oxidoreductase (1b0pA_2).

The primers 5'-CGCGCCGGCACCAGCAGAAACAAAAGCAGAACCAATGACAACC TCGCTGGACGCCTCGATAATCTGGGCCATGATGCAGAATTGAGC-3' (Fw*LINKER*p144) (SEQ ID NO:35) and 5'-GGCCGCTCAATTCTGCATCATGGCCCAGATTATCGAGGCGTCCAGCGAGGTT GTCATTGGTTCTGCTTTTGTTTCTGCTGGTGCCGG-3' (Rv*LINKER*p144) (SEQ ID NO:36) were designed at a 100 mM concentration each. 10 μl of each primer were mixed and hybridized in a thermal cycler: 2 minutes at 95˚C, 10 minutes at 52˚C and taken to 4˚C. The hybridization of these primers is complete in the sequence corresponding to the linker and to p144, but leaves sticky ends compatible with the cleavage by AscI in 5', and compatible with NotI in 3'.

The plasmid pCMV-mApoA1-AscI-p144 was digested with AscI (Buffer 4, New England Biolabs), the DNA was purified, with PerfectPrep DNA Cleanup (Eppendorf, Germany), and it was subsequently digested with NotI (Buffer 3 and BSA, New England Biolabs), due to the incompatibility of digestion buffers. A 1% agarose gel was migrated, the open vector was purified with PerfectPrep DNA Cleanup (Eppendorf, Germany). The product was ligated in a 1:3 (vector: insert) ratio using T4 DNA ligase High Concentration and 2X Rapid Ligation Buffer (Promega Madison, W1, USA) as a buffer solution, incubating the mixture for 10 minutes at room temperature. Top10 bacteria (Invitrogen, Carlsbed, CA) were subsequently transformed. The transformed bacteria were selected by their growth in Petri dishes with LB medium with ampicillin, since the vector contains a gene resistant to this antibiotic. The resulting 6373-nucleotide plasmid will hereinafter be called pCMV-mApoA1-AscI-LINKER-p144. Finally, the sequence was confirmed by means of sequencing.

## 2. Experiments:

### 2.1 Animals:

The experiments have been conducted in female immunocompetent BALB/c and C57BL/6 mice between 5-7 weeks (Harlan, Barcelona, Spain). The animals were treated according to the indications and ethical rules of the Centro de Investigación Médica Aplicada (CIMA, Pamplona, Spain), under specific external pathogen-free conditions.

### 2.2 Animal handling and tumor models:

Each DNA plasmid (20 μg) was resuspended in 1.8 ml of 0.9% saline (Braun) introduced through the tail vein by means of a hydrodynamic injection (Liu et al., 1999, Gene Ther., 6:1258-1266), using 27.5G needles and 2.5ml syringes (Becton-Dickinson, Spain). Blood samples were obtained by a retro-orbital route, after anesthesia by inhalation of isoflurane (Forane, Abbott). The serum was recovered by means of two consecutive centrifugations at 9.1xg for 5 minutes and stored at -20˚C. Parenteral anesthesia was carried out by a 200 μl/mouse intraperitoneal injection with a 9:1 mixture of ketamine (Imalgene) and xylazine (Rompun). The temperatures were measured by abdominal contact with the ThermoKlinik thermometer (Artsana, Grandate, Italy).

#### 2.2.1 Blood analysis:

Blood was extracted from mice to tubes with 0.5% heparin (Mayne) as final concentration. To determine: i) total white blood cells, a 1:1000 dilution of the whole blood was performed in vessels with 20ml of Isoton II Diluent solution and 3 drops of Zap-Oglobin II Lytic Reagent were added 2 minutes before their measurement, ii) total red blood cells, a 1:50000 dilution of the whole blood was performed in vessels with 10 ml of Isoton II Diluent solution, iii) platelets, the whole blood was diluted 1:25 in a tube with 500μl of Isoton II Diluent solution, centrifuged for 1.5 minutes 600g at 4˚C, and supernatant was transferred in a 1:400 dilution to a vessel with 20 ml of Isoton II Diluent the samples were analyzed in a Z1 Coulter Particle Counter with the settings recommended for each case by the manufacturer (all the material and reagents were from Beckman Coulter).

#### 2.2.2 Vaccination models against a CT26

To analyze the anti-tumor efficacy of the gene transfer, two vaccination protocols were carried out:

A) A vaccinations protocol was carried out with 50 μg/mouse of peptide AH-1 with the amino acid sequence SPSYVYHQF (SEQ ID NO:54) (Proimmune Ltd., Oxford, United Kingdom) dissolved in 100 μl/mouse of 0.9% physiological saline and with 100 μl/mouse of Freund's incomplete adjuvant (IFA, SIGMA, Madrid, Spain). The mixture was sonicated in Branson SONIFIER 250. Each animal was vaccinated with 200 μl of the mixture with a 25G needle and a 1 ml syringe (Becton-Dickinson, Spain), of which 100 μl were introduced in the left flank of the mice and 50 μl in the sole. Seven days later, the different constructs were administered by means of a hydrodynamic injection (Liu et al., 1999, Gene Ther., 6:1258-1266). Seven days after the hydrodynamic injection, tumors were established by means of the subcutaneous injection, with insulin syringes (Becton-Dickinson, Spain), of $5 \times 10^5$ CT26 colon adenocarcinoma cells resuspended in 200 μl of HBSS (Gibco-BRL, Paisley, UK) in the right flank of syngeneic BALB/c mice.

B) The gene constructions were administered by means of hydrodynamic injection. One day after the hydrodynamic injection, the vaccination with peptide AH-1 was carried out as previously described. After nine days, $5 \times 10^6$ colorectal adenocarcinoma cells (CT26) were inoculated by means of the subcutaneous injection. The tumor follow-up was carried out with a digital precision gage.

2.3 Detailed Description of cell lines used:

The CT26 cell lines is derived from a BALB/c mouse colorectal adenocarcinoma and was introduced by the carcinogen N-nitroso-N-methyl-urethane, cultured in complete RPMI-1640 medium (Gibco-BRL, Paisley, UK), supplemented with 10% fetal calf serum (FCS) inactivated at 56˚C, 2 mM glutamine, 100 U/ml streptomycin, 100 mg/ml penicillin, 1% 5x10$^{-3}$ β-mercaptoethanol. The cell lines MC38 (murine adenocarcinoma cells), L929 (cells derived from mouse fibroblasts) and 293 (embryonic human kidney cells stably transfected with the E1 region belonging to the human adenovirus type 5, ECACC no. 85120602) cultured in complete DMEM (supplemented with 10% fetal calf serum (FCS) inactivated at 56˚C, 2 mM glutamine, 100 U/ml streptomycin, 100 mg/ml penicillin).

The cells described were cultured in humidified incubating chambers at 37˚C and in a 5% CO2 atmosphere. The culture bottles and plates were from Greiner Bio-one (Essen, Germany).

2.4 Determination of mIFNα1, IFNγ, and Neopterin:

The mIFNα1 levels were measured by ELISA in NUNC maxisorp flat 96-well plates. The anti-mIFNα1 neutralizing Ab antibody (RMMA-1, PBL) was diluted 1/1000 in PBS1x, it was plated 100μl/well and incubated O/N at 4˚C in a humid atmosphere. After five washings in PBS 1x-0.1% Tween-20 (pH 7.2-7.4), the plate was blocked with 300 μl/well of the PBS 1x 1% BSA solution for 1 hour at room temperature. The serum samples were diluted 1/100 in PBS 1x 1% BSA solution and incubated for 1 hour at room temperature. After five washings in PBS 1x-0.1% Tween-20, it was incubated for 1 hour with 100μl/well of rabbit anti-IFNα polyclonal antibody (PBL), diluted 1/1000 in PBS 1x 1% BSA solution. After five washings in PBS 1x-0.1% Tween-20, 100 μl/well of HRP-conjugated donkey α-rabbit IgG (Southern Biotech, Birmingham, CA, USA) were added, dilution 1/4000 in PBS 1x 1% BSA solution, 1 hour at room temperature. After five washings in PBS 1x-0.1% Tween-20, 100 μl/well of BD OptEIA substrate solution (BD) were added, after 15 minutes at room temperature and in darkness, 50 μl of 2N $H_2SO_4$ were added. Finally, the absorbance at 450 nm was measured, and it was corrected at 540 nm.

The IFNγ levels in serum were measured with IFN-γ ELISA Set (BD Biosciences, San Diego, CA). The neopterin levels in serum were measured with Neopterin ELISA (IBL, Hamburg), according to the instructions provided by the manufacturer.

2.5 Quantitative PCR

2 μl of cDNA were incubated with the specific primers of Table 1 using iQ SYBR Green Supermix (Bio-Rad, Hercules, CA). Murine actin was used to standardize the gene expression, because its expression is not affected by mIFNα1 or mApoA1. The mRNA values were represented by the formula $2^{\Delta Ct}$, where $\Delta C_t$ indicates the difference in the threshold cycle between mActin and the target genes.

Table 1. List of primers used.

| Name | Sequence | SEQ ID NO: |
|---|---|---|
| FwATGmApoA1 | 5'-ATGAAAGCTGTGGTGCTGGC-3' | 20 |
| RvTGAmApoA1 | 5'-TCACTGGGCAGTCAGAGTCT-3' | 21 |
| Few AscI mApoA1 | 5'-CCAGGCGCGCCGGATGAACCCCAGTCCCAATG-3' | 27 |
| RvAscImApoA1 | 5'-GGCGCGCCCTGGGCAGTCAGAGTCTCGC-3' | 24 |
| RvSPmApoA1 | 5'-TTGCTGCCATACGTGCCAAG-3' | 30 |
| RvmApoA1p17 | 5'-TCACGCACGCTCATACCAAGAACTCCTAGG AATAAACCAAATACGCTTGGGCGCGCCCTGGGC-3' | 28 |
| RvmApoA1p144 | 5'-TCAATTCTGCATCATGGCCCAGATT ATCGAGGCGTCCAGCGAGGTGGGCGCGCCCTGGGC-3' | 29 |
| RvSPmApoA1p17 | 5'-TCACGCACGCTCATACCAAGAACT CCTAGGAATAAACCAAATACGCTTTTGCTGCCAGAAATGCCG-3' | 31 |

(continued)

| Name | Sequence | SEQ ID NO: |
|---|---|---|
| RvSPmApoA1p144 | 5'-TCAATTCTGCATCATGGCCCAGATTATCGA GGCGTCCAGCGAGGTTTGCTGCCAGAAATGCCG-3' | 32 |
| FwMMp9Asclp144 | 5'- CCAGGCGCGCCGCTTTTCCCGACGTCTACCTCGCTGGACGCCTC- 3' | 33 |
| RvMMp9Asclp144 | 5'-TCAATTCTGCATCATGGCCCA-3' | 34 |
| FwLINKERp144 | 5'-CGCGCCGGCACCAGCAGAAACAAAAGCAGAACCA ATGACAACCTCGCTGGACGCCTCGATAATCTGGGCCATGATGCAGA ATTGAGC-3' | 35 |
| RvLINKERp144 | 5'-GGCCGCTCAATTCTGCATCATGGCCCAGA TTATCGAGGCGTCCAGCGAGGTTGTCATTGGTTCTGCTTTTGTTTC TGCTGGTGCCGG-3' | 36 |
| FwATGmIFNa1 | 5'-ATGGCTAGGCTCTGTGCTTT-3' | 22 |
| RvTGAmIFNa1 | 5'-TCATTTCTCTTCTCTCAGTC-3' | 23 |
| FwAsclmIFNa1 | 5'-GGCGCGCCCTGTGACCTGCCTCAGACTCA-3' | 25 |
| RvAsclmIFNa1 | 5'-GGGCGCGCCTTTCTCTTCTCTCAGTCTTC-3' | 26 |
| FwUSP18 | 5'-CCAAACCTTGACCATTCACC-3' | 37 |
| RvUSP18 | 5'-ATGACCAAAGTCAGCCCATCC-3' | 38 |
| FwISG15 | 5'-GATTGCCCAGAAGATTGGTG-3' | 39 |
| RvISG15 | 5'-TCTGCGTCAGAAAGACCTCA-3' | 40 |
| FwIRF1 | 5'-CCAGCCGAGACACTAAGAGC-3' | 41 |
| RvIRF1 | 5'-CAGAGAGACTGCTGCTGACG-3' | 42 |
| FwMx1 | 5'-ATCTGTGCAGGCACTATGAG-3' | 43 |
| RvMx1 | 5'-CTCTCCTTCTTTCAGCTTCC-3' | 44 |
| FwmActin | 5'-CGCGTCCACCCGCGAG-3' | 45 |
| RvmActin | 5'-CCTGGTGCCTAGGGCG-3' | 46 |
| qPCR FwmIFNa | 5'-TCTYTCYTGYCTGAAGGAC-3' | 47 |
| qPCR RvmIFNa | 5' CACAGRGGCTGTGTTTCTTC-3' | 48 |
| Few 2-5 OAS | 5'- ACTGTCTGAAGCAGATTGCG-3' | 49 |
| Rv 2-5 OAS | 5'- TGGAACTGTTGGAAGCAGTC-3' | 50 |

2.6 *In Vivo* Killing

Female BALB/c mice (N=3/group) were immunized by means of a hydrodynamic injection as has been previously described, on day 0 with 20μg of *pCMV-LacZ and with* 20μg of each construction to study: (i) *pCMV-mApoA1* ii) *pCMV-IFNα1* iii) *pCMV-mApo-IFN* iv) *pCMV-mIFN-ApoA1* dissolved in 0.9% physiological saline (Braun). On day 7, splenocytes from non-immunized BALB/c mice spleen were isolated and the red blood cells were lysed with ACK solution (Cambrex, Walkersville, MD). The obtained splenocytes were divided into two groups and one of them was incubated for 30 minutes at 37°C with RPMI 1640 medium and 9 $\mu$M of peptide TPHPARIGL (cytotoxic epitope derived from $\beta$-galactosidase, Proimmune Ltd., Oxford, United Kingdom). The second group received the same treatment without the peptide. The splenocytes loaded with the peptide were labeled with 2.5 $\mu$M CFSE (CFSE[high]) (Molecular Probes, Eugene, OR). The control splenocytes were labeled with 0.25 $\mu$M CFSE (CFSE[low]). Finally, both populations were mixed in a 1:1 ratio and $10^7$ cells were injected intravenously into naïve mice or into the immunized mice. After 24 hours, the animals were sacrificed. The extracted spleens were broken up and the ratio of CFSE[high] and CFSE[low] cells was analyzed by means of flow cytometry using FACSalibur (Becton Dickinson, Mountain View, Calif, USA). The percentage of specific lysis was calculated according to the following formula:

$$Lysis = 100 - \left( 100 \times \frac{\left( \dfrac{\%CFSE^{high}immunized}{\%CFSE^{low}immunized} \right)}{\left( \dfrac{\%CFSE^{high}control}{\%CFSE^{low}control} \right)} \right)$$

### 2.7 Measurement of expression of SRB1:

Splenocytes from C57BL/6 mice spleen were isolated. The extracted and broken up spleens were divided into 8 groups, of which four groups were incubated for 10 minutes with rabbit anti-SR-B1 polyclonal antibody (Novus Biologicals Littleton, CO) and with the BD Pharmigen antibodies: i) R-PE-Conjugated Rat Anti-Mouse CD4 (L3T4) Monoclonal Antibody to define the CD4+ cell populations and APC-Conjugated Rat Anti-Mouse CD8a (Ly-2) Monoclonal Antibody, to define the CD8+ cell populations. ii) APC-Conjugated Mouse Anti-Mouse NK-1.1 (NKR-P1B and NKR-P1C) Monoclonal Antibody to define the NK cell population. iii) APC-Rat Anti-Mouse CD11b to define the monocyte cell population iv) APC- CD11c to define the dendritic cell population. The other four groups were used as a control, being incubated for 10 minutes with the respective antibodies, without anti-SRB1. The labeled splenocytes were washed in PBS and 5% fetal bovine serum and were incubated for 10 minutes with FITC-Conjugated Donkey Anti-Rabbit IgG Antibody (Jackson ImmunoResearch, West Grove, PA). The samples thus stained were studied by flow cytometry using FACSalibur (Becton Dickinson, Mountain View, Calif, USA).

### 2.8 Isolation of HDLs by differential ultracentrifugation in sodium bromide gradient:

24 hours after the hydrodynamic injection with the plasmids mApoA1, IFN$\alpha$1, Apo-IFN, IFN-Apo or ApoAI-linker-P144, blood was extracted from mice to tubes with 0.5% heparin (Mayne) as final concentration, and the plasma was extracted immediately by centrifugation (5000g, 20 minutes).

The sodium bromide (NaBr) solutions at different densities were prepared in a final volume of 25 mL in distilled water. EDTA at a final concentration of 0.05% (w/v) was added. NaBr (Fluka) was added to obtain the solutions: 0.225 g ($\rho$= 1.006g/ml), 1.431 g ($\rho$= 1.04 g/ml), 7.085 g ($\rho$= 1.21 g/ml) and 13.573 g ($\rho$= 1.4 g/ml). Due to the fact that NaBr is a highly hygroscopic salt, the density was verified and corrected by adding distilled water when necessary.

The method of sequential separation of lipoproteins by flotation after ultracentrifugation was performed with small modifications of the protocol described by Rodriguez-Sureda et al (Analytical Biochemistry 303, 73-77 (2002)) in Ultracentrifuge Optima MAX, with TLA100.4 rotor (Beckman Coulter), from 2-4 mL of mouse plasmas (unified according to the samples) at the following densities: VLDL < 1.006 g/mL, LDL 1.006-1.04 g/mL and HDL 1.04-1.21 g/mL. i) Isolation of VLDL: 400 $\mu$l of mouse plasma were transferred to 3ml polycarbonate tubes and 1100$\mu$l of a $\rho$= 1.006g/ml NaBr solution were added. The samples were centrifuged for 2 hours, 4°C, 336000g. Approximately 650 $\mu$l of supernatant were collected with a pipette and stored at -20°C. ii) Isolation of LDL: The remaining volume of sediment was taken to a density of 1.04 by adding the volume calculated by the formula of the $\rho$= 1.4g/ml NaBr solution: $\rho$ = m/V

V $_{1.4NaBr}$ = V$_{bf}$(d-d$_{bf}$) / 1.4 - d V $_{1.4NaBr}$ = Vol of 1.4 solution to be added

V $_{bf}$ = Vol of the sediment
d $_{bf}$ = density of the sediment

The volume was taken to 1.5 ml with the $\rho$= 1.04g/ml NaBr solution and the samples were centrifuged for 2.5 hours, 4°C, 336000g. 300-400$\mu$l of supernatant were collected with a pipette and stored at -20°C. iii) Isolation of HDL: approximately 800$\mu$l of sediment were transferred to a new tube, and the density was adjusted to 1.21g/mL as has already been described, and taken to a volume of 1.5 mL with the $\rho$= 1.21g/ml NaBr solution. The samples were centrifuged for 3.5 hours, 4°C, 336000g, and a supernatant fraction of approximately 400 ul corresponding to HDL, and the sediment fraction corresponding to the lipoprotein-free plasma (LDP) were collected and stored at -20˚C.

2.9 Electrophoresis and immunoblotting against mApo A1:

25µl of HDL or LDP fraction for each of the samples were separated in 4-20% Trishepes PAGE LongLife iGels (Nusep) gradient gels, transferred to nitrocellulose membrane (Whatman). The protein was detected with the goat polyclonal antibody against mApoA1, 1:200 dilution (Goat polyclonal anti-Apolipoprotein A1, Santa Cruz Biotechnology) and antibody against goat IgG, 1:20000 dilution (Anti-goat IgG (whole molecule)- HRP conjugated, Sigma-Aldrich). The membrane was developed with ECL Plus Western Blotting Detection Reagent (Amersham).

2.10 IFN activity bioassay: Cytopathic effect (CPE):

The IFN activity units of the HDL fractions isolated from mouse plasma containing Apo-IFN and IFN-Apo were calculated by means of an activity bioassay, measuring the capacity of IFN to protect the cells against the cytopathic activity of the encephalomyocarditis lytic virus (EMCV) over a wide range of plated IFN concentrations by the successive dilution of the samples. On this dilution, $3 \times 10^5$ L929 cells/well were plated in 96-well Cellstar cell culture plates (Greiner bio-one) and incubated O/N at 37˚C 5% $CO_2$ to reach the monolayer of adherent cells. Then, the same amount of pfu/well of EMCV was added and incubated for 24 hours until achieving the lysis of the untreated cells used as control. At this point, the viable cells protected by the IFN effect are measured by luminometry with the ViaLight Plus Kit developing solution (Lonza) following the manufacturer's instructions. The reading is plotted to generate a dose-response curve (Prism 5, GraphPad Software, Inc.) from which the potency of the IFN preparations in terms of antiviral activity units can be calculated with reference to the dilutions of a rIFN$\alpha$ recombinant protein standard (PBL) used in each assay.

2.11 Experiments with rIFN and isolated HDL IFN-Apo fractions:

10000 IU of mouse rIFN alpha (CHO derived mouse, Hycult Biotechnol) or 10000 IU of HDL IFN-Apo measured by activity bioassay was retro-orbitally injected into mice.

2.12 Statistical analysis of the data:

The statistical analysis of the data was performed using the Prism 5 computer program (GraphPad Software, Inc.). The tumor appearance data were represented in Kaplan-Meier graphs and analyzed by means of the log-rank test. The data studied at different times was analyzed by means of repeated-measures ANOVA followed by the Bonferroni test. The remaining parameters were analyzed by means of ANOVA and followed by Dunnett's post hoc analysis for carrying out multiple comparisons. $p < 0.05$ values were considered to be significant.

**EXAMPLE 2**

The hydrodynamic administration of the chimeric constructs ApoAI - IFN$\alpha$ increases the serum IFN levels

To compare the levels of serum murine IFN$\alpha$ levels, plasmids expressing apolipoprotein AI (ApoAI), murine interferon alpha 1 (IFN$\alpha$1), Apo-IFN (fusion of ApoAI and IFN$\alpha$1) or IFN-Apo (fusion of IFN$\alpha$1 and ApoAI) are administered to groups of four mice by means of a hydrodynamic injection Sera obtained after 6 hours and on day 1, 3, 6 and 9 were analyzed by means of a sandwich ELISA. The sera of the mice which received the control plasmid expressing ApoAI did not have detectable IFN$\alpha$ levels, indicating that the hydrodynamic administration *per se* or the expression of ApoAI did not induce the expression of the endogenous IFN$\alpha$ (Figure 1). The mice which were injected with the plasmid expressing IFN$\alpha$1 had high IFN$\alpha$ levels after 6 hours and decreased rapidly (Figure 1). The mice which received plasmids encoding Apo-IFN or IFN-Apo had higher serum interferon levels on day 1. Furthermore, high IFN$\alpha$ levels could be detected on day 3, unlike the mice injected with plasmid IFN$\alpha$1 (Figure 1). Therefore, the constructs expressing the IFN$\alpha$ and ApoAI fusion proteins have higher and more sustained serum IFN$\alpha$ levels.

**EXAMPLE 3**

The messenger RNA kinetics does not vary in the chimeric constructs ApoAI - IFN$\alpha$

The difference in the serum IFN$\alpha$ levels can be explained by the increase of the plasma half-life of the fusion proteins with respect to IFN$\alpha$ or by the increase of the expression of these proteins. To distinguish between these two alternatives, the messenger RNA (mRNA) kinetics of these constructs was analyzed. To that end, the livers of the mice which had received a hydrodynamic injection with plasmids expressing ApoAI, IFN$\alpha$1, Apo-IFN and IFN-Apo on day 0, 1, 3 and 6 were collected. The RNA of these samples was extracted and a quantitative RT-PCR was performed. As can be observed in Figure 2, IFN$\alpha$1 mRNA levels were detected on day 1 in the IFN$\alpha$1, Apo-IFN and IFN-Apo samples but not in the ApoAI samples. The mRNA levels did not have significant differences between the groups with any construct with IFN$\alpha$. On day 3 and 6, IFN$\alpha$1 mRNA levels were not detected in any sample. Therefore, the mRNA kinetics is similar in all the groups which received a construct containing IFN$\alpha$1, the hypothesis of the increase of expression in the chimeric constructs ApoA1 - IFN$\alpha$1 being able to be discarded.

**EXAMPLE 4**

The mice injected with the chimeric constructs ApoAI - IFN$\alpha$ have higher serum neopterin and body temperature levels

To verify i) that the chimeric proteins maintained the biological activity of IFN$\alpha$ and ii) that the more sustained levels were correlated with a higher biological activity, two parameters which increase after the administration of IFN$\alpha$ were analyzed. These parameters were studied three days after the administration of plasmids, at which time IFN$\alpha$ produced by the construct with IFN$\alpha$1 was no longer detected but that produced by the chimeric constructs was detected. Firstly, the serum neopterin levels were analyzed. Neopterin is a catabolite product of GTP, synthesized by the macrophages stimulated by type I and II interferons. The three plasmids containing the IFN$\alpha$ sequence increased the serum neopterin levels but only the chimeric constructs increased significantly (Figure 3 A). Secondly, the body temperature in the abdominal area of the injected mice was measured. High levels were observed with the three constructs, the levels obtained after the administration of the chimeric constructs being emphasized (Figure 3 B). Therefore, the chimeric proteins are capable of increasing two biological parameters induced by interferon, it being demonstrated that they retain the biological activity and that this activity is correlated with the serum IFN$\alpha$ levels on day 3.

**EXAMPLE 5**

The chimeric constructs ApoAI - IFN$\alpha$ increase the hepatic expression levels of interferon-stimulated genes

The activity of the type I interferons is mediated by proteins encoded by interferon-stimulated genes (ISGs). After IFN$\alpha$ binds to their membrane receptor, a signaling cascade is activated which results in the activation of ISG transcription. To verify if the chimeric constructs also induce these genes, the mRNA levels of four of these genes was analyzed on day 3 after the hydrodynamic administration. The genes which were analyzed are IRF1, 2'-5' OAS, USP18, ISG15, Mx1. As can be observed in Figure 4, the chimeric constructs increase the mRNA levels of the studied ISGs. An increase induced by the plasmid expressing IFN$\alpha$ can also be detected despite the fact that on day 3, serum IFN$\alpha$ levels were no longer detected.

**EXAMPLE 6**

The constructs with IFN$\alpha$ increase the number and the activation or splenocytes

To explore the immunostimulating activity of the constructs, the increase of the number and activity of spleen cells was analyzed first. To that end, the plasmids were injected by means of a hydrodynamic injection and six days later, the spleens were broken up, the total cells were counted and after labeling the splenocytes with antibodies to identify the main lymphocyte populations and with an activation marker (CD69), they were analyzed by means of flow cytometry. The injection of constructs with IFN$\alpha$ significantly increased the number of splenocytes. The construct encoding for IFN-Apo is considerably emphasized in this assay (Figure 5A). To label different splenocyte populations, anti-CD4 antibodies were used as a CD4$^+$ T cell marker; anti-CD8 as CD8$^+$ T cell marker; anti-CD19, as a B cell marker; and anti-CD49b, as an NK cell marker. In relation to CD4$^+$ T cells, the chimeric constructs increased the percentage of activated CD4$^+$ cells, unlike IFN$\alpha$ (Figure 5B). However, the IFN$\alpha$ did increase the percentage of CD8$^+$ cells although in a non-significant manner. The increase was greater and significant with Apo-IFN and especially high with IFN-Apo (Figure 5C). The percentage of activated B cells follows the same profile as that of CD8$^+$. In this case, only IFN-Apo caused a significant increase (Figure 5D). Finally, the NK cells gave a high activation with the IFN$\alpha$ plasmid, this parameter not being exceeded by the chimeric constructs (Figure 5E). This data suggests that IFN-Apo can have a potent adjuvant effect.

**EXAMPLE 7**

IFN-Apo increases the specific lysis induced by cytotoxic lymphocytes

To verify the adjuvant effect of IFN-Apo, the increase of the cytotoxic activity induced by a DNA vaccine was analyzed in presence of the different constructs. The LacZ gene encoding the immunogenic β-galactosidase protein was chosen as an antigen model. The plasmid encoding this protein was injected together with plasmids encoding ApoA1, IFNα1, Apo-IFN or IFN-Apo. Seven days after the gene vaccination, splenocytes labeled with 2.5 μM CFSE and loaded with the cytotoxic epitope H2Kd TPHPARIGL, derived from the β-galactosidase protein, were intravenously injected. As an internal control, splenocytes were injected with 0.25 μM CFSE without peptide. After twenty-four hours, the specific lysis of the splenocytes loaded with the cytotoxic epitope was quantified by flow cytometry. In Figure 6, a higher lysis is observed with respect to ApoAI with IFNα, followed by Apo-IFN and by IFN-Apo, the construct with which the highest values of specific lysis are obtained. These results were correlated with the results of the percentage of CD8+ T cell activation and allow concluding that IFN-Apo is the construct with the highest adjuvant effect in a gene vaccination model.

**EXAMPLE 8**

Expression of SR-BI

The increase in the adjuvant activity can be due to the increase in the stability of IFNα or due to the fact that the ApoAI fraction of IFN-Apo allows a higher interaction of IFNα with immune system cells. To explore the latter hypothesis, the presence of the main receptor for ApoAI, SR-BI, was analyzed in different immune system populations. Splenocytes from a naive mouse were isolated and labeled with an anti-SRB-I antibody and with an antibody for defining the population. The following antibodies were used: anti-CD4, as a marker of CD4+ T cell marker; anti-CD8, as a CD8+ T cell marker; anti-CD49b, as an NK cell marker; anti-CD11b, as a monocytes/macrophages marker; and anti-CD 11c, as a dendritic cell marker. The SRB-I receptor was detected in all the analyzed populations. In the immune system effector cells (CD4+ T, CD8+ T and NK cells), the percentage of cells expressing this receptor ranges between 15% and 28%. This percentage rises up to more than 50% in cells with antigen-presenting capacity such as monocytes and dendritic cells (Figure 7). This result suggests that one of the possible mechanisms for increasing the adjuvant capacity can be a higher maturation of the antigen-presenting cells.

**EXAMPLE 9**

IFN-Apo improves the efficacy of an antitumor vaccination protocol.

After demonstrating that IFN-Apo has a higher adjuvant activity than IFN, it was evaluated if this effect translates into a higher antitumor efficacy in a vaccination protocol. To that end, BALB/c mice received a hydrodynamic injection with the Apo, IFN or IFN-Apo plasmids and on the following day they were vaccinated with the cytotoxic peptide AH-1 in Freund's incomplete adjuvant. This peptide is presented by the CT26 tumor line, which was inoculated into the different experimental groups 9 days after the vaccination. Most of the mice of the control group, which received the vaccination plus the hydrodynamic injection with the Apo plasmid, developed a subcutaneous tumor in the inoculation site of the CT26 tumor cells. Mice which received IFN in addition to the vaccination present a behavior which does not differ significantly from that of the control group. However, about 60% of the mice which received the vaccination and the IFN-Apo plasmid were capable of rejecting the tumor cells and did not present tumors throughout the 30 days of the experiment (Figure 8). Therefore, the greater adjuvant effect of IFN-Apo causes an increase of the efficacy of a vaccination protocol.

**EXAMPLE 10**

IFN-Apo presents lower hematological toxicity than IFN.

One of the limitations of the IFN is its considerably hematological toxicity, which can lead to the suspension of the treatment in certain patients who develop an intense leukopenia and/or thrombocytopenia. The evolution was analyzed then, after the hydrodynamic administration of the different constructs, of the leukocytes and platelets in blood. It is observed in Figure 9 A that all the constructs having interferon presented low blood levels of leukocytes on day 1 after the hydrodynamic administration of the plasmids. This early effect can be mediated by a blocking of the exit of leukocytes from the secondary lymphoid organs described for IFN (Shiow LR et al. Nature. 440(7083):540-4 (2006)). However, on day 3, when the toxic effect due to the antiproliferative properties of IFN can be seen, only the mice treated with IFN presented low levels. In the mice treated with IFN-Apo, blood leukocytes recovered their normal levels. Regarding the platelets (Figure 9 B), a decrease was observed on day 3 in those animals treated with IFN. This decrease was significantly lower in the mice treated with IFN-Apo. Therefore, IFN-Apo reduces the decrease induced by IFN both of leukocytes and of platelets.

**EXAMPLE 11**

IFN-Apo increases the interferon-induced genes in the brain less than IFN.

Another of the main adverse effects of the IFN, which limits its use in certain patients, is neuropsychiatric disorders. To study the effect of the new fusion molecules in the central nervous system, BALB/c mice were injected with the plasmids encoding the different constructs and after 24 hours, the mice were sacrificed and their brain extracted. The increase in the interferon-induced genes (ISGs) in the different groups (Figure 10) was analyzed by means of quantitative RT-PCR. Although the plasma levels of the fusion proteins are higher than those of IFN (Figure 1), the increase of ISGs was significantly greater in IFN than in the IFN-Apo molecules. This data indicates that the fusion of the Apo molecule to IFN modifies the blood-brain barrier (BBB) passage. 0.02%-0.18% of the plasma interferon alpha traverses the BBB by means of passive diffusion (Greig, N.H., et al. J Pharmacol Exp Ther, 245(2): 574-80 (1988); Greischel, A., et al. Arzneimittelforschung, 38(10): 1539-43 (1988); Smith, R.A., et al. Clin Pharmacol Ther. 37(1): 85-8. (1985)). Therefore, the concentration at the brain level will be proportional to the plasma concentration. In contrast, the BBB passage of HDLs occurs by means of active transport mediated by SR-BI, very controlled and low levels being maintained (Karasinska, J.M., et al. J Neurosci. 29(11): 3579-89 (2009)). Our results suggest that the binding of biologically active compounds to the apolipoprotein AI forces these chimeric molecules to follow the mechanism of transport through the BBB of HDLs.

**EXAMPLE 12**

The IFN-Apo fusion protein circulates incorporated into high density lipoproteins (HDLs).

About 97% of the apolipoprotein AI present in the blood, circulate in the form of a macromolecular lipoprotein complex called high density lipoproteins. To study if the fusion protein was capable of being incorporated into HDLs, 24 hours after injecting the plasmids encoding IFN and the IFN-Apo molecules by hydrodynamic route, the HDLs were isolated from the serum by means of differential centrifugation in NaBr gradient. An IFN bioassay , i.e., an assay of protection from the cytopatic effect of a virus, was performed with these fractions, in which cells previously incubated with the samples with IFN in serial dilutions are compared with a cytopathic virus. If there is interferon in the samples, the virus will not be capable of lysing the pretreated cells. The HDLs obtained from mice which received the plasmid encoding IFN were not capable of protecting the cells from the cytopatic effect of the encephalomyelitis virus, indicating that IFN does not circulate bound to the HDLs. In contrast, the two IFN-Apo molecules can indeed be detected by this technique in the HDLs (Figure 11 A). Then, a western blot was performed to detect apolipoprotein AI in the HDLs-free (HDLs -) serum fractions and the fraction of HDLs (HDLs+) of each group experimental. Apolipoprotein AI was not detected in any HDL-depleted fraction, indicating the correct isolation of the HDLs. Both in the group which received the Apo plasmid, and in the group which received that of IFN, only one band was detected in the fraction of HDLs, corresponding to the height of the endogenous apolipoprotein AI. In contrast, a band with a greater height was detected in the group with the Apo-IFN molecule, corresponding to the fusion molecule. In the case of the IFN-Apo molecule, two bands were detected in addition to the endogenous ApoAI, indicating the formation of dimers in part of the chimeric protein (Figure 11 B). This phenomenon can be due to the fact that the C terminal end is free in this construct, allowing the interaction with other ApoAI molecules. This data indicates that the fusion molecules are capable of being incorporated in high density lipoproteins. Therefore, the biodistribution of these molecules will be governed by the laws ruling the biodistribution of HDLs, which can explain, at least partially, the drastic change observed in some of the IFN activities.

**EXAMPLE 13**

The re-administration of HDLs containing IFN-Apo maintains the properties observed after the hydro dynamic administration.

The possibility of purifying HDLs containing IFN-Apo from the sera of mice to which the plasmid encoding IFN-Apo was administered allows providing physiological IFN-Apo nanolipoparticles to study the properties both *in vitro* and *in vivo* thereof. HDL with IFN-Apo was purified and the equivalent to 10000 IU of IFN per mouse was administered. On day 3, the leukocyte and platelet count in blood was analyzed. The dose administered of recombinant IFN was not capable of causing a decrease of these parameters. But the mice which received the HDLs of IFN-Apo presented significantly higher levels (Figure 12 A). This phenomenon can be due to the fact that IFN-Apo stimulates the proliferation of latent hematopoietic cells more efficiently than IFN (Essers MA, et al. Nature. Feb 11 (2009)). On day 1, the depression state induced by IFN was determined in these mice. Again, there is a significant difference between the mice which received recombinant IFN and those which received an equivalent dose of HDLs of IFN-Apo, the data obtained after the hydrodynamic administration being reproduced.

**EXAMPLE 14**

The ApoAI-linker-P144 construct increases IL12-mediated IFNγ induction

Interleukin 12 (IL12) is an immunostimulating cytokine with a potent antitumor activity. Its activity is essentially mediated by IFNγ. The production of this mediator is regulated by TGFβ, therefore its blocking by means of the inhibitor peptides p17 or p144 can increase IFNγ induction and, therefore, the antitumor activity of IL12. To study if chimeric constructs formed by ApoAI and the TGFβ inhibitor peptides, bound by means of different peptide sequences, can increase I112-mediated IFNγ induction, a plasmid encoding murine IL12 and plasmids encoding the different constructs were administered by means of a hydrodynamic injection. ApoAI was used as a control. Two constructs were generated with p17: i) spP17, containing the sequence encoding peptide p17 preceded by the ApoAI signal peptide, the release of peptide p17 to the extracellular medium being achieved. ii) ApoAI-P17, containing the gene encoding ApoAI followed by three binding amino acids (GAP), and the sequence encoding p17. The constructs spP 144 and ApoAI-P144 were generated with p 144, substituting the sequence encoding p 17 with that of p144. Another two constructs were furthermore generated: i) ApoAI-MMP9-P144, containing a target for metalloproteinase 9 (MMP9) as a binding peptide. ii) ApoAI-linker-P144, containing a sequence with extended conformation as a binding peptide.

Four days after the hydrodynamic injection, the serum IFNγ levels were analyzed by means of ELISA. The injection of plasmids encoding for IL12 and ApoAI generated detectable IFNγ levels. The injection of the constructs with p17 did not increase these levels (Figure 13A). However, the administration of the construct generating p144 significantly increased the IFNγ levels (Figure 13B). The construct ApoAI-linker-P144 generated the highest levels, significantly greater than those induced by p144 alone (Figure 13B). Curiously enough, the constructs ApoAI-P144 and ApoAI-MMP9-P144 did not increase IFNγ induction, indicating that the binding peptide sequence can have a great influence in the activity of the chimeric construct. The construct ApoAI-MMP9-P144 is an example of a latent inhibitor which would only be active in the presence of MMP9, which upon cleaving the sequence binding to ApoAI will release the active peptide p144 in the site in which MMP9 is expressed. This protease is expressed by many types of tumors, including hepatocarcinomas, and by myeloid suppressor cells, which invade the tumor stroma. Therefore, this construct will allow releasing p144 in the site in which it has to mainly act, limiting the adverse effects of systemic TGFβ inhibition.

**EXAMPLE 15**

The constructs expressing p17 and ApoAI-linker-P144 increase the percentage of tumor-free vaccinated mice

To verify the biological activity of the constructs with the TGFβ inhibitor peptides in an independent experimental model, BALB/c mice were vaccinated with the cytotoxic epitope H2Kd AH1 (SPSYVYHQF, SEQ ID NO:54) with Freund's incomplete adjuvant. Seven days later, the different constructs were administered by means of a hydrodynamic injection. After another seven days, $5 \times 10^5$ CT26 cells were inoculated subcutaneously. The percentage of tumor-free animals was analyzed over time. In the group which had received the vaccine and the control construct expressing ApoAI, all the mice developed a subcutaneous tumor in the CT26 cell inoculation site. However, more than 50% of the mice which had received one of the two constructs expressing p17 remained tumor-free at the end of the experiment (Figure 14A). In the case of the constructs with p144, the construct expressing peptide p144, the construct ApoAI-P144 and the construct ApoAI-MMp9-P144 had a very limited effect, the experiment ending with less than 20% tumor-free mice. Surprisingly, the construct ApoAI-linker-P144 was capable of preventing the onset of tumors in more than 85% of the mice.

**EXAMPLE 16**

The ApoAI-linker-P144 protein circulates incorporated into high density lipoproteins

To study if the ApoAI-linker-P144 protein forms complexes with HDLs, the fraction containing the HDLs was isolated from a serum of a mouse to which the plasmid encoding the ApoAI-linker-P144 was administered by hydrodynamic route. To that end the serum was subjected to a differential centrifugation in NaBr gradient. Once the HDLs were purified, a western blot was performed to detect apolipoprotein AI. In addition to the major band corresponding to the endogenous apolipoprotein AI, a band with a greater height corresponding to the ApoAI-linker-P144 molecule was detected (Figure 15). Therefore, the apolipoprotein AI having fused therapeutic peptides is capable of being incorporated and circulating in the form of a physiological nanolipoparticle.

**EXAMPLE 17**

HDLs containing ApoAI-linker-P144 increase the IFNγ induced by IL-12.

The purification of HDLs containing ApoAI-linker-P144 allows obtaining physiological nanolipoparticles with the capacity to inhibit TGFβ activity. To show their *in vivo* activity, HDLs purified from an animal expressing ApoAI-linker-P144 were inoculated into mice to which a hydrodynamic injection with a plasmid expressing interleukin 12 in response to the administration of doxycycline is simultaneously administered. As positive control, the plasmid ApoAI-linker-P144 was coadministered. The IFNγ levels obtained after the administration of the HDLs are similar to those obtained after the hydrodynamic injection (Figure 16). In both cases, they are significantly higher than those obtained after the induction of the IL-12 plasmid without the presence of a TGFβ inhibitor. Therefore, peptide P144 present in HDLs is capable of blocking TGFβ *in vivo*, allowing a greater induction of IFNγ. Therefore, the incorporation of peptides into HDLs through their fusion with the apoliprotein AI represents an attractive strategy for formulating novel therapeutic peptides.

**Claims**

1. A conjugate comprising

(i) an Apo A molecule or a functionally equivalent variant thereof and

(ii) a compound of therapeutic interest

wherein components (i) and (ii) are covalently bound.

2. A conjugate according to claim 1, wherein the Apo A molecule is selected from the group of ApoA-I, ApoA-II, ApoA-III, ApoA-IV and ApoA-V or a functionally equivalent variant thereof.

3. A conjugate according to claims 1 or 2, wherein the Apo A molecule is selected from the group of human and murine Apo A.

4. A conjugate according to any of claims 1 to 3, wherein component (ii) is a polypeptide.

5. A conjugate according to claim 4, wherein components (i) and (ii) form a single polypeptide chain.

6. A conjugate according to claim 5, wherein the C-terminal end of component (i) is bound to the N-terminal end of component (ii) or wherein the N-terminal end of component (i) is bound to the C-terminal end of component (ii).

7. A conjugate according to any of claims 4 to 6, wherein component (ii) is an interferon.

8. A conjugate according to claim 7, wherein the interferon is human or mouse interferon $\alpha$1 or interferon $\alpha$5.

9. A conjugate according to any of claims 4 to 6, wherein component (ii) is a TGF-beta inhibitor.

10. A conjugate according to claim 9, wherein the TGF-beta inhibitor is selected from the group of SEQ ID NO: 4 (P144) and SEQ ID NO: 5 (P17) or functionally equivalent variants thereof.

11. A conjugate according to any of claims 4 to 10, wherein components (i) and (ii) are connected by a peptide linker.

12. A conjugate according to claim 11, wherein the peptide linker is a flexible peptide and/or contains a protease recognition site.

13. A conjugate according to claim 12, wherein the linker is selected from the group of SEQ ID NO:13 (APAETKAEPMT), GAP or a matrix metalloprotease-9 recognition site (SEQ ID NO:19).

14. A polynucleotide or a gene construct comprising a polynucleotide encoding a polypeptide according to any of claims 5 to 13.

15. A vector comprising a polynucleotide or a gene construct according to claim 14.

16. A host cell comprising a polynucleotide or a gene construct according to claim 14 or a vector according to claim 15.

17. A nanolipoparticle comprising a conjugate according to any of claims 1 to 14.

18. A nanolipoparticle according to claim 17 wherein said nanolipoparticle is a high density lipoprotein (HDL)

19. A pharmaceutical preparation comprising a therapeutically effective amount of a conjugate according to any of claims 1 to 13, a polynucleotide or a gene construct according to claim 14, a vector according to claim 15, a host cell according to claim 16 or a nanolipoparticle according to claims 17 or 18 and a pharmaceutically acceptable carrier or excipient.

20. A conjugate according to any of claims 1 to 13, a polynucleotide or a gene construct according to claim 14, a vector according to claim 15, a host cell according to claim 16, a nanolipoparticle according to claims 17 or 18 or a pharmaceutical preparation according to claim 19 for its use in medicine.

21. A conjugate according to any of claims 1 to 13, a polynucleotide or a gene construct according to claim 14, a vector according to claim 15, a host cell according to claim 16, a nanolipoparticle according to claims 17 or 18 or a pharmaceutical preparation according to claim 19 for the treatment of liver diseases or of diseases associated with the immune system.

22. A conjugate according to any of claims 1 to 13, a polynucleotide or a gene construct according to claim 14, a vector according to claim 15, a host cell according to claim 16, a nanolipoparticle according to claims 17 or 18 or a pharmaceutical preparation according to claim 19 for the treatment of a disease selected from the group of chronic hepatitis C, chronic hepatitis B, hepatocarcinoma, Parkinson's disease, acute intermittent porphyria, pulmonary fibrosis, bone metastasis, systemic sclerosis, morphea, skin cancer, actinic keratosis, keloid scars, bums, cardiac fibrosis, renal fibrosis, viral infections, bacterial infections, parasitic infections, rheumatoid arthritis and Non-Hodgkin's lymphoma.

23. A conjugate according to any of claims 1 to 13, a polynucleotide or a gene construct according to claim 14, a vector according to claim 15, a host cell according to claim 16, a nanolipoparticle according to claims 17 or 18 or a pharmaceutical preparation according to claim 19 for its use as a vaccine adjuvant, as an adjuvant in immunotherapy, as an adjuvant in the treatment of colon cancer, as an antiangiogenic drug, as a hepatic or renal protector.

24. A combination comprising

(a) a first component selected from the group of a conjugate according to any of claims 1 to 13, a polynucleotide or a gene construct according to claim 14, a vector according to claim 15, a host cell according to claim 16, a nanolipoparticle according to claims 17 or 18 or a pharmaceutical preparation according to claim 17, wherein component (ii) is a TGF-$\beta$1 inhibitor peptide and
(b) a second component selected from the group of an immunostimulatory cytokine, a polynucleotide encoding said cytokine, a vector comprising said polynucleotide, a TGF-$\beta$1 inhibitory peptide, a cytotoxic agent or a combination thereof.

25. A combination according to claim 24, wherein the TGF-$\beta$1 inhibitor peptide in component (a) or in component (b) is selected from the group of peptide p144 and peptide p17.

26. A combination according to claims 24 or 25, wherein the immunostimulatory cytokine in component (b) is IL-12.

27. A combination according to any of claims 24 to 26 for the treatment of cancer.

FIG. 1

FIG. 2

FIG. 3

FIG. 4

**A**

FIG. 5

FIG. 5

D

E

FIG. 5

FIG.6

FIG. 7

**C**

**D**

FIG. 7

FIG. 7

FIG. 8

FIG.9

FIG. 10

FIG. 11

EP 2 305 309 A2

FIG. 12

EP 2 305 309 A2

FIG. 13

FIG. 14

FIG. 15

FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 07130873 A
- WO 02086091 A
- WO 200473684 A
- WO 04082720 A
- WO 8702061 A
- WO 07021494 A
- US 5762923 A
- US 5766582 A
- WO 200031135 A
- WO 200519244 A
- WO 2007048857 A
- WO 9807751 A
- WO 9811140 A
- WO 07023476 A
- WO 9525786 A
- WO 8702062 A
- US 20050255042 A
- WO 04054622 A
- WO 06107617 A
- WO 07046893 A
- WO 07112193 A
- US 6809186 B
- WO 2006135436 A
- WO 07069090 A

**Non-patent literature cited in the description**

- **Lou et al.** *World J. Gastroenterol.,* 2005, vol. 11, 954-959
- **Kim et al.** *Molecular Therapy,* 2007, vol. 15, 1145-1152
- **Kramer et al.** *J.Biol.Chem.,* 1992, vol. 267, 18598-18604
- **Monaco et al.** *EMBO J.,* 1987, vol. 6, 3253-3260
- **Altschul, S. et al.** BLAST Manual. NCBI NLM NIH Bethesda
- **Altschul, S. et al.** *Mol. Biol.,* 1990, vol. 215, 403-410
- **Eisenberg, S. et al.** *J.Lipid Res.,* 1973, vol. 14, 446-458
- **Blum et al.** *J. Clin. Invest.,* 1977, vol. 60, 795-807
- **Graversen et al.** *J Cardiovasc Pharmacol.,* 2008, vol. 51, 170-177
- **Pestka, S. ; Krause, C.D. ; Walter, M.R.** *Immunol Rev.,* 2004, vol. 202, 8-32
- **Le Bon A. et al.** *Nat. Immunol.,* 2003, vol. 4, 1009-1015
- **Le Bon A. et al.** *J. Immunol.,* 2006, vol. 176, 4682-4689
- **Le Bon A. et al.** *J Immunol.,* 2006, vol. 176, 2074-8
- **Bekisz et al.** *Growth Factors,* 2004, vol. 22, 243-251
- **Petska et al.** *Immunological Reviews,* 2004, vol. 202, 8-32
- **Sambrook ; Russel.** Molecular Cloning: to Laboratory manual. Cold Spring Harbor, 2001
- **Muller, K.M. ; Arndt, K.M. ; Alber, T.** *Meth. Enzymology,* 2000, vol. 328, 261-281
- **Nielsen, B.B. et al.** *FEBS Lett.,* 1997, vol. 412, 388-396
- **Pack P. ; Pluckthun, A.** *Biochemistry,* 1992, vol. 31, 1579-1584
- **Jackson et al.** *Biochim Biophys Acta,* 1976, vol. 420, 342-349
- **Borresen, A.L. ; Kindt, T.J.** *J. Immunogenet.,* 1978, vol. 5, 5-12
- **Forgez, P ; Chapman, M.J.** *J. Biochem. Biophys. Methods,* 1982, vol. 6, 283-96
- **Feng et al.** *Protein. Expr. Purif.,* 2006, vol. 46, 337-42
- **Pyle et al.** *Biochemistry,* 1996, vol. 35, 12046-52
- **Brissette et al.** *Protein Expr. Purif.,* 1991, vol. 2, 296-303
- **Bonen, D.K.** *J. Biol. Chem.,* 1997, vol. 272, 5659-67
- **Miyatake et al.** *J. Virol,* 1997, vol. 71, 5124-32
- **Sandig et al.** *Gene Ther.,* 1996, vol. 3, 1002-9
- **Arbuthnot et al.** *Hum.GeneTher,* 1996, vol. 7, 1503-14
- **Wang, L. et al.** *Proc. Natl. Acad. Sci. USA,* 1997, vol. 94, 11563-11566
- **Lerch et al.** *Vox Sang,* 1996, vol. 71, 155-164
- **C. Faulí i Trillo.** Tratado de Farmacia Galénica. S.A. de Ediciones, 1993
- Remington's Pharmaceutical Sciences. Williams & Wilkins PA, 2000
- **Templeton.** *DNA Cell Biol.,* 2002, vol. 21, 857-867

- **Lindgren, A. et al.** *Trends Pharmacol. Sci,* 2000, vol. 21, 99-103
- **Schwarze, S.R. et al.** *Trends Pharmacol. Sci.,* 2000, vol. 21, 45-48
- **Lundberg, M et al.** *Mol. Therapy,* 2003, vol. 8, 143-150
- **Snyder, E.L. ; Dowdy, S.F.** *Pharm. Res.,* 2004, vol. 21, 389-393
- **Liu, F. et al.** *Gene Ther,* 1999, vol. 6, 1258-66
- **Liu et al.** *Science,* 1999, vol. 305, 1437-1441
- **Hodges et al.** *Exp.Opin.Biol.Ther,* 2003, vol. 3, 91-918
- **Brodeur et al.** *J. Bone Miner Res.,* 2008, vol. 23, 326-37
- **Yeh et al.** *Atherosclerosis,* 2002, vol. 161, 95-103
- **Cai, S. F. et al.** *J. Lipid Res.,* 2001, vol. 42, 902-909
- **Miquel et al.** *Gut.,* 2003, vol. 52, 1017-1024
- **Acton et al.** *J.Biol.Chem.,* 1994, vol. 269, 21003-21009
- **Liu et al.** *Gene Ther.,* 1999, vol. 6, 1258-1266
- **Rodriguez-Sureda et al.** *Analytical Biochemistry,* 2002, vol. 303, 73-77
- **Shiow LR et al.** *Nature,* 2006, vol. 440 (7083), 540-4
- **Greig, N.H. et al.** *J Pharmacol Exp Ther,* 1988, vol. 245 (2), 574-80
- **Greischel, A. et al.** *Arzneimittelforschung,* 1988, vol. 38 (10), 1539-43
- **Smith, R.A. et al.** *Clin Pharmacol Ther.,* 1985, vol. 37 (1), 85-8
- **Karasinska, J.M. et al.** *J Neurosci.,* 2009, vol. 29 (11), 3579-89
- **Essers MA et al.** *Nature,* 11 February 2009